# EUROPEAN PATENT APPLICATION

(11) **EP 4 799 583 A1**
(43) Date of publication of application: **02.09.2026**
(21) Application number: 26160763.4
(22) Date of filing: 25.02.2026
(51) Int. Cl.: A61B 34/20

(54) **SYSTEMS AND METHODS FOR AUTOMATIC ORIENTATION OF ASYMMETRICAL IMPLANTS AND ASYMMETRICAL END EFFECTORS**

(30) Priority: 27.02.2025 US 202563764051 P
(71) Applicant: Stryker European Operations Limited, Carrigtwohill, Co. Cork T45 HX08 (IE)
(72) Inventor: GUTH, Yannik, 79100 Freiburg (DE); MARTINS, Maria Manuel Carvalho de Freitas, 68420 Eguisheim (FR)
(74) Representative: Röthinger, Rainer

(57) **Abstract**

Systems, methods, and software for displaying an asymmetrical implant relative to image data representative of a patient, wherein the asymmetrical implant is configured to be attached to an implant insertion tool in one of multiple orientations. A computing device receives the image data representative of the patient, receives tracking data indicative of a pose of the patient and a pose of the implant insertion tool, and receives a model of the asymmetrical implant which includes an implant pose vector. The computing device determines an orientation of the implant pose vector relative to the image data based on the tracking data. The computing device presents a representation of the model of the asymmetrical implant relative to a representation of the implant insertion instrument based on the determined orientation of the implant pose vector.

## Description

### RELATED APPLICATIONS

The present application claims priority to and all the benefits of U.S. Provisional Patent Application No. 63/764,051, filed on February 27, 2025, the entire contents of which are expressly incorporated herein by reference.

### BACKGROUND

Surgical navigation systems assist users in tracking/locating objects in an operating room. For instance, navigation systems assist surgeons in placing surgical instruments relative to a patient's anatomy. Typically, the tool and the anatomy are tracked together with their relative movement shown on a display. Often the navigation system includes tracking devices attached to the object being tracked, and a localizer determines positions of the tracking devices to determine a position and/or orientation of the object. The navigation system then monitors movement of the objects via the tracking devices, such as by determining the location of these tracking devices relative to a coordinate system associated with the localizer. During surgery, a surgeon often uses instruments that are inserted into the body of a patient. Once inside the patient's body, the surgeon loses vision of the tip of the instrument. In order to help the surgeon navigate the instrument in such a case, a surgical navigation system can be used to track the instrument and provide visual, tactile, and/or acoustic guidance to the surgeon. One way to track the instrument is to attach a tracker onto the instrument. A camera in the operating room detects the tracker and generates data that is used to calculate the position of the tracker and, therefore, the instrument. Commonly, the patient is also tracked, which enables calculating the position of the instrument relative to the patient.

In many cases, implants and/or end effectors are coupled to the surgical instruments and navigated together with the instruments. This way, the pose of the implant and/or end effector may be shown relative to the patient on the display. For example, a spinal implant, or an end effector formed integrally with a trial implant, may be coupled to an introducer and guided between adjacent vertebrae of the patient while being tracked and shown on the display. The implants and end effectors often lack trackable features, and the surgical navigation systems often rely on known spatial relationships to determine the pose of the implant or end effector. Issues may arise, however, if the implant and/or end effector is asymmetric and can be coupled to the instrument in multiple orientations.

### SUMMARY

According to a first aspect, a computer-implemented method is provided for displaying an asymmetrical implant relative to image data representative of a patient, the asymmetrical implant being configured to be attached to an implant insertion instrument in one of multiple orientations. The method includes receiving the image data representative of the patient, receiving tracking data indicative of a pose of the patient and a pose of the implant insertion instrument, and receiving a model of the asymmetrical implant which includes an implant pose vector, determining an orientation of the implant pose vector of the model relative to the image data based on the tracking data and an expected relationship between the asymmetrical implant and the patient, and displaying a representation of the model of the asymmetrical implant relative to a representation of the implant insertion instrument based on the determined orientation of the implant pose vector.

According to a second aspect, a computer-implemented method is provided for displaying an asymmetrical implant relative to image data representative of a patient, the asymmetrical implant being configured to be attached to an implant insertion instrument in one of multiple orientations. The method includes providing an instrument model associated with the implant insertion instrument, the instrument model including an x-vector and a z-vector, providing an implant model associated with the asymmetrical implant, the implant model including an implant pose vector, providing a patient model associated with the patient, the patient model includes a patient pose vector, and determining a pose of the implant insertion instrument relative to the patient. The method further includes displaying a representation of the patient, a representation of the instrument model, and a representation of the implant model oriented such that the implant pose vector is in a first orientation relative to the x and z axes of the instrument model. In order to do so, the method includes determining an expected relationship between the implant pose vector and the patient pose vector of the patient model, and adjusting the displayed representation of the implant model relative to the instrument model such that the implant pose vector in is a second orientation relative to the x and z axes based on the expected relationship between the implant pose vector and the patient pose vector of the patient model.

According to a third aspect, a computer-implemented method is provided for provided for displaying an asymmetrical end effector relative to image data representative of a patient, the asymmetrical end effector being configured to be attached to an instrument in one of multiple orientations. The method includes receiving the image data representative of the patient, receiving tracking data indicative of a pose of the patient and a pose of the instrument, receiving a model of the asymmetrical end effector which includes an end effector pose vector, determining an orientation of the end effector pose vector of the model based on the tracking data and an expected relationship between the asymmetrical end effector and the patient, and displaying a representation of the model of the asymmetrical end effector relative to a representation of the instrument based on the determined orientation of the end effector pose vector.

According to a fourth aspect, a computer-implemented method is provided for displaying an asymmetrical end effector relative to image data representative of a patient, the asymmetrical end effector being configured to be attached to an instrument in one of multiple orientations. The method includes providing an instrument model associated with the instrument, the instrument model including an x-vector and a z-vector, providing an end effector model associated with the asymmetrical end effector, the end effector model including an end effector pose vector, providing a patient model associated with the patient, the patient model includes a patient pose vector, and determining a pose of the instrument relative to the patient. Additionally, the method includes displaying a representation of the patient, a representation of the instrument model, and a representation of the end effector model oriented such that the end effector pose vector is in a first orientation relative to the x and z axes of the instrument model, determining an expected relationship between the end effector pose vector and the patient pose vector of the patient model, and adjusting the displayed representation of the end effector model relative to the instrument model such that the end effector pose vector in is a second orientation relative to the x and z axes based on the expected relationship between the end effector pose vector and the patient pose vector of the patient model.

According to a fifth aspect, a computer-implemented method is provided for correcting visualization of a cage implant in a surgical navigation application, the method comprising: receiving patient image data associated with a patient coordinate system; receiving a cage model of a cage implant with a thick side and an opposing thin side, the cage model defining a cage axis that points from the thick side towards the thin side; determining, from the patient image data, a posterior axis that points toward a posterior side of the patient in the patient coordinate system; tracking an orientation of the cage model relative to the patient coordinate system; detecting that the cage axis and the posterior axis are oppositely directed; and in response to detecting that the cage axis and the posterior axis are oppositely directed, rotating the cage model by approximately 180 degrees within the navigation application to align the cage axis with the posterior axis, thereby correcting the visualization.

According to a sixth aspect, a computer-implemented method is provided for displaying an implant in a surgical navigation environment, the method comprising: receiving image data representative of a patient; receiving a model of an asymmetrical implant; determining an implant axis associated with the model; determining, from the image data, an anatomical axis associated with the patient; determining an orientation of the implant axis relative to the anatomical axis; and automatically adjusting a displayed orientation of the model based on the determined orientation to ensure the implant axis is aligned with the anatomical axis.

Also provided are a surgical system comprising a tracking system and a computing system (or one or more controllers) configured to implement any features of the computer-implemented methods of any aspect above; and a non-transitory computer readable medium or computer program product comprising instructions, which when executed by one or more processors, are configured to implement any features of the computer-implemented methods of any aspect above. Any of the above aspects can be combined in part or in whole with any other aspect. Any of the above aspects, whether combined in part or in whole, can be further combined with any of the following implementations, in full or in part.

Depending on the implementation, representations of the instrument, implant, and/or patient may be displayed based on various factors. In some implementations, the representation of the implant insertion instrument may be displayed relative to a representation of the patient based on the tracking data.

In one implementation, the asymmetrical implant can be a cage implant, e.g., for spinal procedures such as for interbody fusion to correct lordotic deformities. The cage implant can have a wedge/angled/tapered shaped body with a first end with a first thickness/width and an opposing second end with a second thickness/width that is thicker/wider than the first. The wedge/angled/tapered shaped body makes the orientation of the implant clinically relevant because the cage should be oriented so that the second (thicker) end of the wedge is positioned anteriorly (toward the front of the body) and the first (thinner) end of the wedge is positioned posteriorly (toward the back of the body). The implant body can have one or more openings or slots formed within the body to allow bone growth through the body. The cage implant can attach to the implant insertion instrument (e.g., cage inserter) via a coupling interface that enables the cage implant to be mounted in at least two different orientations.

The orientation of the implant pose vector may be determined in multiple ways. For example, the method may include defining a patient pose vector relative to the image data based on the tracking data, and determining the orientation of the implant pose vector of the model based on an expected relationship between the implant pose vector and the patient pose vector. The patient pose vector may be defined relative to the image data based on the tracking data and a patient registration transform. Determining the orientation of the implant pose vector of the model may include aligning the implant pose vector relative to the patient pose vector. In some implementations, the implant pose vector is oriented in the same direction as the z-vector of the instrument model in the first orientation. Additionally, where the x-vector of the instrument model is perpendicular to the z-vector of the instrument model, the second orientation of the implant pose vector may be rotated about the x-vector relative to the first orientation of the implant pose vector. The implant pose vector may be oriented opposite of the z-vector of the instrument model in the second orientation.

In some implementations, the orientation of the implant pose vector may be realized as a first orientation of the implant pose vector relative to a z-vector of the representation of the implant insertion instrument. In such an implementation, the method may further include displaying the representation of the model of the asymmetrical implant in the first orientation relative to the representation of the implant insertion instrument as the implant insertion instrument is moved relative to the patient. Further, the method may include determining an alignment metric based on the implant pose vector and a patient pose vector as the implant insertion instrument is moved relative to the patient, determining a second orientation of the implant pose vector of the model relative to the z-vector of the representation of the implant insertion instrument which is different from the first orientation, and displaying the representation of the model of the asymmetrical implant in the second orientation relative to the representation of the implant insertion instrument based on the alignment metric. Even further, the alignment metric may be an angle formed by the implant pose vector and the patient pose vector, and the method may include defining a misalignment threshold, selecting one of the first orientation and the second orientation based on the misalignment threshold and the alignment metric, and displaying the representation of the model of the asymmetrical implant in the selected one of the first orientation and the second orientation. The second orientation of the implant pose vector may be rotated about an x-vector of the representation of the implant insertion instrument relative to the first orientation of the implant pose vector.

The orientation of the end effector pose vector may be determined in multiple ways. For example, the method may include defining a patient pose vector relative to the image data based on the tracking data, and determining the orientation of the end effector pose vector of the model based on an expected relationship between the end effector pose vector and the patient pose vector. The patient pose vector may be defined relative to the image data based on the tracking data and a patient registration transform. Determining the orientation of the end effector pose vector of the model may include aligning the end effector pose vector relative to the patient pose vector. The end effector pose vector may be oriented in the same direction as the z-vector of the instrument model in the first orientation. Additionally, where the x-vector of the instrument model is perpendicular to the z-vector of the instrument model, and the second orientation of the end effector pose vector may be rotated about the x-vector relative to the first orientation of the end effector pose vector. The end effector pose vector may be oriented opposite of the z-vector of the instrument model in the second orientation.

In some implementations, the orientation of the end effector pose vector may be realized as a first orientation of the end effector pose vector relative to a z-vector of the representation of the instrument. In such an implementation, the method may further include displaying the representation of the model of the asymmetrical end effector in the first orientation relative to the representation of the instrument as the instrument is moved relative to the patient. Additionally, the method may include determining an alignment metric based on the end effector pose vector and a patient pose vector as the instrument is moved relative to the patient, determining a second orientation of the end effector pose vector of the model relative to the z-vector of the representation of the instrument which is different from the first orientation, and displaying the representation of the model of the asymmetrical end effector in the second orientation relative to the representation of the instrument based on the alignment metric. Further, the alignment metric may be an angle formed by the end effector pose vector and the patient pose vector. In such cases, the method may further include defining a misalignment threshold, selecting one of the first orientation and the second orientation based on the misalignment threshold and the alignment metric, and displaying the representation of the model of the asymmetrical end effector in the selected one of the first orientation and the second orientation. The second orientation of the end effector pose vector may be rotated about an x-vector of the representation of the instrument relative to the first orientation of the end effector pose vector.

In some implementations, the representation of the instrument model is displayed relative to the representation of the patient model based on the pose of the instrument relative to the patient, and the representation of the end effector model is displayed relative to the representation of the instrument model.

### BRIEF DESCRIPTION OF THE DRAWINGS

Advantages of the present disclosure will be readily appreciated as the same becomes better understood by reference to the following detailed description when considered in connection with the accompanying drawings.
Figure 1 shows an exemplary configuration of a surgical suite for performing a medical procedure on a patient using the surgical system.
Figures 2A and 2B illustrate a surgical instrument of one implementation with a tracking device and implant coupled thereto.
Figures 3A and 3B depict the implant of Figures 2A and 2B in different orientations relative to the surgical instrument of Figures 2A and 2B.
Figure 4 shows an exemplary configuration of a graphical user interface of a surgical navigation system according to one implementation.
Figure 5 shows representations of the instrument and implant of Figure 1 overlaid onto image data of a patient.
Figure 6 includes a flowchart describing a method for displaying representations of an instrument and an implant relative to image data of a patient according to one implementation.
Figures 7A and 7B illustrate an implant representation being reoriented relative to an instrument representation according to the method of Figure 6.
Figures 8A and 8B show a surgical instrument according to another implementation.
Figure 9 includes a flowchart describing a method for displaying representations of an end effector relative to image data of a patient according to one implementation.
Figures 10A and 10B illustrate an end effector representation being reoriented relative to image data according to the method of Figure 9.
Figures 11A-11D depict reorienting a representation of an implant relative to image data of a patient according to one implementation.
Figure 12 includes a flowchart describing a method for displaying representations of an instrument and an implant relative to image data of a patient according to one implementation.
Figure 13 includes a flowchart describing a method for displaying representations of an end effector relative to image data of a patient according to one implementation.

### DETAILED DESCRIPTION

Referring to the Figures, wherein like numerals indicate like or corresponding parts throughout the several views, a surgical system 100 including a surgical navigation system 110 and methods for using the same are shown throughout.

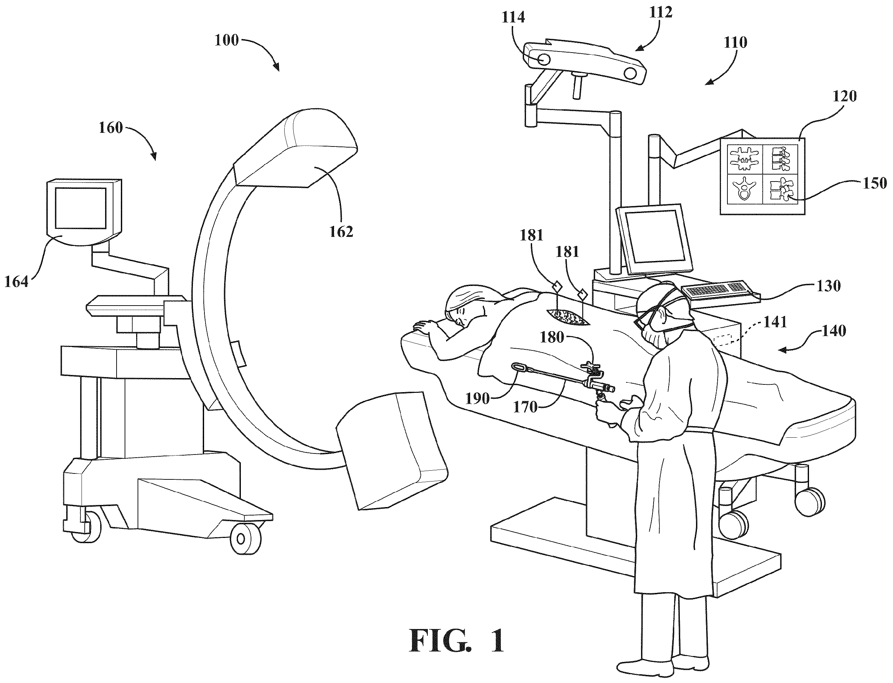

Referring to FIG. 1, an exemplary configuration of a surgical suite for performing a medical procedure on a patient using the surgical system 100 is shown. The surgical navigation system 110 may include a tracking system or localizer 112, a display device 120, user input devices 130, a navigation computer 140, and a controller 141 to facilitate communication between the navigation computer 140, the remainder of the navigation system 110, and optionally other elements of the system 100. The navigation computer 140 may include a processor (not shown), a memory device (not shown), and a storage device (not shown). The navigation computer 140 may be a personal computer, laptop computer, tablet computer, or any other suitable computing device. The navigation computer 140 may include surgical navigation software including one or more modules and/or operating instructions related to the operation of the surgical navigation system 110 and to implement the various routines, functions, or methods disclosed herein. Further, the controller 141 may be part of the navigation computer 140, or the controller 141 may be implemented by multiple computing devices, such as the navigation computer 140 and a cloud computing device. In some implementations, the controller 141 includes a cloud computing device which communicates with the navigation computer 140 (*e.g.,* the processor of the navigation computer 140). The techniques described herein may be implemented by any one or more controllers or processors implemented by any one or more of the devices/computers/servers described herein.

The display device 120 is configured to display various graphical user interfaces (GUI) 150 and patient images (e.g., pre-operative patient images or intraoperative patient images). The pre-operative images may be uploaded to the surgical navigation system 110 prior to the surgical procedure. A user such as a medical professional may interact with the various GUIs 150 via user input devices 130, via touch input, or via other suitable means. The display 120 of the surgical navigation system 110 may be configured to display various prompts or data entry boxes. For example, the display 120 may be configured to display a text box or prompt that allows the user to manually enter or select the type of surgical procedure to be performed.

The display 120 may be further configured to display a surgical plan for a medical procedure overlaid on the patient images. The surgical plan may include the surgical pathway for executing the medical procedure, planned trajectory, orientation, and/or position for the medical instrument and/or implant during the medical procedure. The surgical plan may also include a pose of an implant or medical device to be inserted during the medical procedure overlaid onto the patient data or image. It is contemplated that the surgical navigation system 110 may be configured to display and/or project a holographic image of surgical pathway for executing the medical procedure or planned trajectory or orientation for the medical instrument during the medical procedure. This may include projecting the surgical pathway onto the patient or other surface in the operating room. It may also include a projection of the surgical pathway onto the head unit worn by the user, such as a lens, shield, or glasses of the head unit. An exemplary configuration of the surgical navigation system 110 including a display worn by the user to display the target trajectory and/or target location is disclosed in International Publication No. WO/2018/203304 A1, the entirety of which is hereby incorporated by reference.

In some implementations, the display device 120 may be configured as an extended reality device configured to execute any of the graphical functions described herein. The extended reality device can be implemented by a hand-held device (e.g., tablet or smart phone) or a head-mounted device. The extended reality device may be configured to superimpose, overlay, or combine any of the described computer-generated graphics with real-world views to implement an extended reality, augmented reality, and/or mixed reality experience for the user. The real-world views may be views may be those acquired directly by the eyes of the user or may be a real-world video stream captured by one or more cameras of the extended reality device. When a head-mounted device is utilized, the head-mounted device may comprise a transparent lens or one or more display screens positioned directly in front of the eyes of the user to display the computer-generated graphics relative to the real-world views.

The GUI 150 may be configured to allow the user to input or enter patient data or modify the surgical plan. The patient data, in addition to the patient images, may include additional information related to the type of medical procedure being performed, the patient's anatomical features, the patient's specific medical condition, and/or operating settings for the surgical navigation settings. For example, in performing a spinal fusion procedure, the user may enter information via the user input devices 130 and/or the GUI 150 related to the specific vertebra or vertebra on which the medical procedure is being performed. The user may also input various anatomical dimensions related to the vertebrae and/or the size and shape of a medical device or implant to be inserted during the medical procedure. The user input devices 130 and/or the GUI 150 may also be configured to allow the user to select, edit or manipulate the patient data. For example, the user may identify and/or select anatomical features from the patient data. This may include selecting the surgical site, such as selecting the vertebra and/or specific area on the vertebra where the medical procedure is to be performed.

The surgical navigation system 110 may be configured to utilize segmentation to facilitate various features of surgical navigation, such as tool guidance and the generation of alert zones of interests around critical anatomical features. These critical anatomical features may include cortical walls, nerves, blood vessels, or similar critical anatomical structures. The alert zones may be defined by one or more virtual boundaries. The user may also provide input to the user input devices 130 or to the GUI 150 to identify additional critical anatomical features and/or alert zones in addition to what was suggested by the controller 141 or wish to edit alert zones and/or virtual boundaries generated by the controller 141. The user may also provide input to the user input devices 130 or to the GUI 150 to select and/or input a target location, target trajectory, target depth, or similar feature of the surgical pathway to help guide the user in performing the medical procedure. The input to the user input devices 130 or to the GUI 150 may be provided to select the surgical instrument to be used, to select the device and/or implant to be inserted, to select a planned pose where the device or implant is to be placed within the patient, and to allow the user to select the parameters of the implant to be inserted, such as the length and/or diameter of the screw to be inserted.

The surgical system 100 may also include an imaging system 160 in communication with the surgical navigation system 110. The imaging system 160, such as CT or MRI imaging device, may perform intraoperative imaging. If the imaging system 160 is a CT imaging device, the imaging system 160 may generate CT image data. The imaging system 160 may include a scanner 162 and an imager display 164. The scanner 162 may be utilized to take an image of the patient and display it on the imager display 164. For example, the scanner 162 may include a C-arm configured to be rotated about the patient to produce a plurality of images of the patient. The imaging system 160 may also include a processor (not shown) including software, as is known by those skilled in the art, which is capable of taking the plurality of images captured by the scanner 162 and producing a two-dimensional image and/or a three-dimensional model of at least a portion of the patient. The imager display 164 may be configured to display the resulting two-dimensional image and/or three-dimensional model.

The imaging system 160 may also be in communication with the controller 141 of the surgical navigation system 110. The imaging system 160 may be configured to communicate via a wired and/or a wireless connection with the controller 141. For example, the imaging system 160 may be configured to provide pre-operative and/or intra-operative image data, such as the resulting 2D image and/or 3D model of the patient, to the controller 141 to provide the resulting 2D image and/or 3D model to the display 120. If the imaging system 160 is a CT imaging device, the imaging system 160 may provide the controller 141 with CT image data.

The surgical system 100 may also include a surgical instrument 170, a tracking device 180 coupled to the instrument 170, and an implant 190 coupled near a distal end of the surgical instrument 170. In the illustrated implementation of FIG. 1, the surgical instrument 170 is an implant insertion instrument configured to assist the user in implanting a spinal implant between adjacent vertebrae of the patient. The tracking device 180 may include a plurality of markers that are capable of being identified and/or tracked by the surgical navigation system 110. Reliable tracking of surgical instruments during the execution of surgical procedures to follow the planned surgical pathway and/or to avoid critical anatomical structures is of the utmost importance. Furthermore, providing feedback and/or notifying the user executing the procedure when the surgical instrument becomes misaligned with the surgical pathway and/or is at risk of impinging on a critical anatomical structure is of similar importance.

The navigation system 110 may utilize the localizer 112 to track the instrument assembly 170, the surgical robot, and/or other elements of the surgical system 100. The localizer 112 may also be used to track the patient. The localizer 112 may track the poses of the instrument assembly 170, the surgical robot, other elements of the surgical system 100, and/or the patient relative to a localizer coordinate system 113. For example, the localizer 112 may include one or more sensors 114 for tracking the tracking device 180 coupled to the surgical instrument 170 and the patient tracker 181 coupled to the patient. The sensors may include cameras, such as CCD cameras, CMOS cameras, and/or optical image cameras, magnetic sensors, radio frequency sensors, or any other sensor adapted to detect and/or sense the position of a tracking device 180 of the surgical instrument assemblies 170. Description of a suitable localizer, and the various localizers that it can utilize may be found in U.S. Patent Publication No. 2017/0333137, which is hereby incorporated by reference in its entirety.

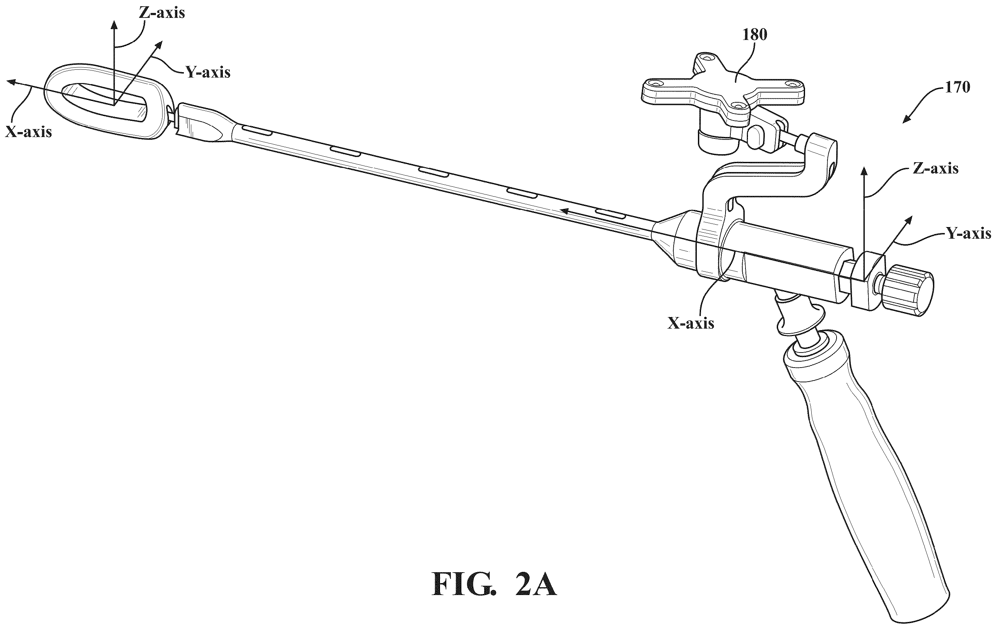

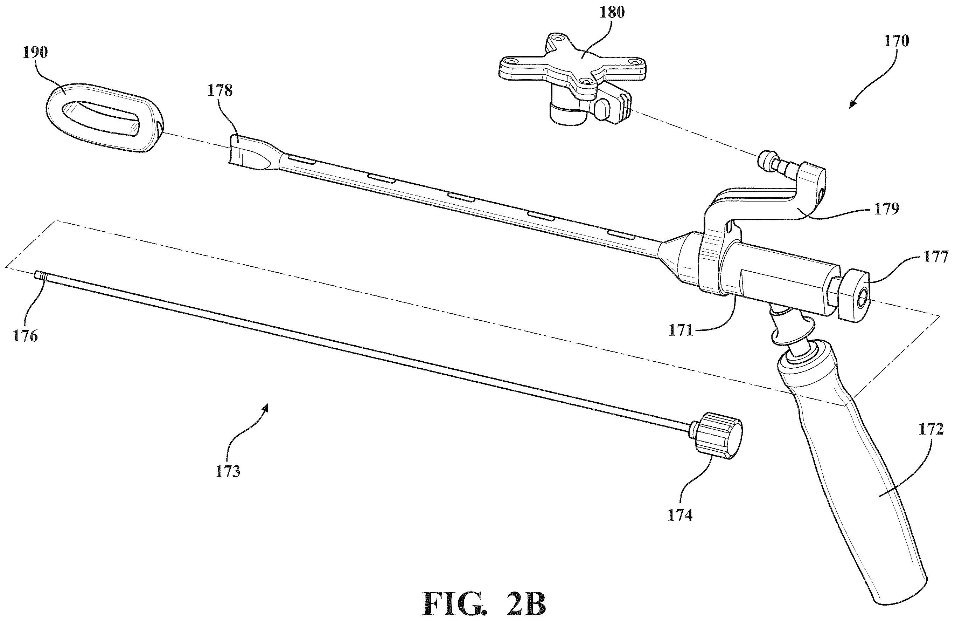

Referring to FIGS. 2A and 2B, the surgical instrument 170 is shown in more detail. In the illustrated implementation, the surgical instrument 170 is an implant insertion instrument. As best shown in FIG. 2B, the surgical instrument 170 may include an instrument body 171, a handle 172 coupled to the instrument body 171, and an implant coupling mechanism 173 configured to be received by the instrument body 171 and to provide attachment means for the implant 190 to be coupled to the instrument 170. In one implementation, the implant coupling mechanism 173 includes a knob 174, a shaft 175 extending from the knob 174, and a threaded portion 176 disposed at a distal end of the shaft 175 opposite the knob 174. The instrument body 171 includes a proximal end 177 and a distal end 178 and defines a bore extending from the proximal end 177 to the distal end 178. The instrument 170 may also include a tracking mount, such as a tracking post 179 configured to removably couple the tracking device 180 to the instrument body 171. The tracking post 179 may be disposed between the proximal and distal ends 177, 178 and extend from the instrument body 171.

During use, the implant coupling mechanism 173 is inserted through the proximal end 177 of the instrument body 171 via the bore defined by the instrument body 171. Once inserted, the threaded portion 176 extends from the distal end 178 of the instrument body 171. Subsequently, while bringing the implant 190 into abutment with the distal end 178 of the instrument body 171, the knob 174 may be rotated so that the threaded portion 176 engages complementary threads of the implant 190 to removably couple the implant 190 to the instrument 170. Then, the navigation system 110 may be used to track the pose of the instrument 170, and the implant 190 coupled thereto, by tracking the tracking device 180.

As shown in FIG. 2A and described in more detail below, the navigation system 110 may associate an instrument coordinate system 182 with the instrument 170, the instrument coordinate system 182 including intersecting X, Y, and Z axes. Additionally, the navigation system 110 may associate an implant coordinate system 184 with the implant 190, the implant coordinate system 184 including intersecting X, Y, and Z axes. The navigation system 110 may then track the poses of the instrument and implant coordinate systems 182, 184 relative to the localizer coordinate system 113 by tracking the instrument tracker 180.

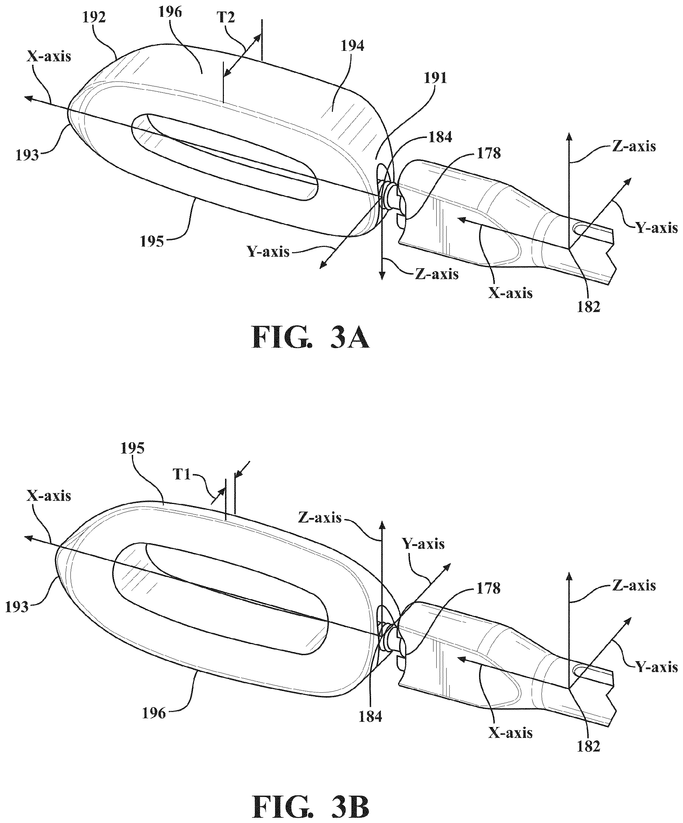

Referring to FIGS. 3A and 3B, the implant 190 is shown from multiple perspectives. In the illustrated implementation, the implant 190 is an asymmetrical interbody implant configured to be implanted between two adjacent vertebrae of a spine. The implant 190 includes a first side 195 and a second side 196. The first side 195, also referred to herein as the thin side, has a first thickness T1, and the second side 196, also referred to herein as the thick side, has a second thickness T2 which is larger than the first thickness T1. Further, the implant 190 includes a first end 191 and a second end 193, at least one of which defines complementary threads configured to cooperate with the threaded portion 176 of the coupling mechanism 173 to couple the implant 190 to the instrument 170.

During navigated spine surgery, the pose of the instrument 170 may be determined by the navigation system 110 based on a tracked pose of the tracking device 180 coupled to the instrument 170 and a known spatial relationship between the tracking device 180 and at least a portion of the instrument 170, such as the distal end 178 of the instrument 170. Further, the position of the implant 190, while coupled to the instrument 170, may be determined based on a known spatial relationship between the implant 190 and the instrument 170. That said, the implant 190 may be coupled to the instrument 170 in multiple orientations. For example, as shown in FIG. 3A, the implant 190 may be coupled to the instrument 170 such that the Z-axis of the implant coordinate system 184 is oriented opposite of the Z-axis of the instrument coordinate system 182. Further, as shown in FIG. 3B, the implant 190 may be coupled to the instrument 170 so that the Z-axis of the implant coordinate system 184 is oriented in the same direction as the Z-axis of the instrument coordinate system 182.

In cases where the implant 190 is asymmetrical, such as the implant 190 of the illustrated implementation, the implant coordinate system 184 may need to be correctly oriented relative to the instrument coordinate system 182 (and the localizer coordinate system 113 by extension) to permit the navigation system 110 to accurately track the pose of the implant 190 relative to the patient. Some methods of ensuring that the pose of the implant 190 is correctly tracked relative to the pose of the instrument 170 exist but are otherwise unsatisfactory for various reasons. For example, U.S. Patent No. 12,076,093 to Brainlab AG, filed on September 30, 2020, provides one such method. In this example, the method includes determining a calibrated virtual model of a cage implant by holding a tip of a tracked pointing device against various positions of the cage implant. The cage implant is then tracked according to the calibrated virtual model. Methods like that provided by the aforementioned patent require a manual process to be carried out by the user/surgeon require additional tools, such as a tracked pointing device, and may be time consuming and/or tiresome for the surgeon to carry out during a surgical operation. In light of the disadvantages of existing methods, an automatic method of determining the pose of the implant 190 relative to the instrument 170 is described below.

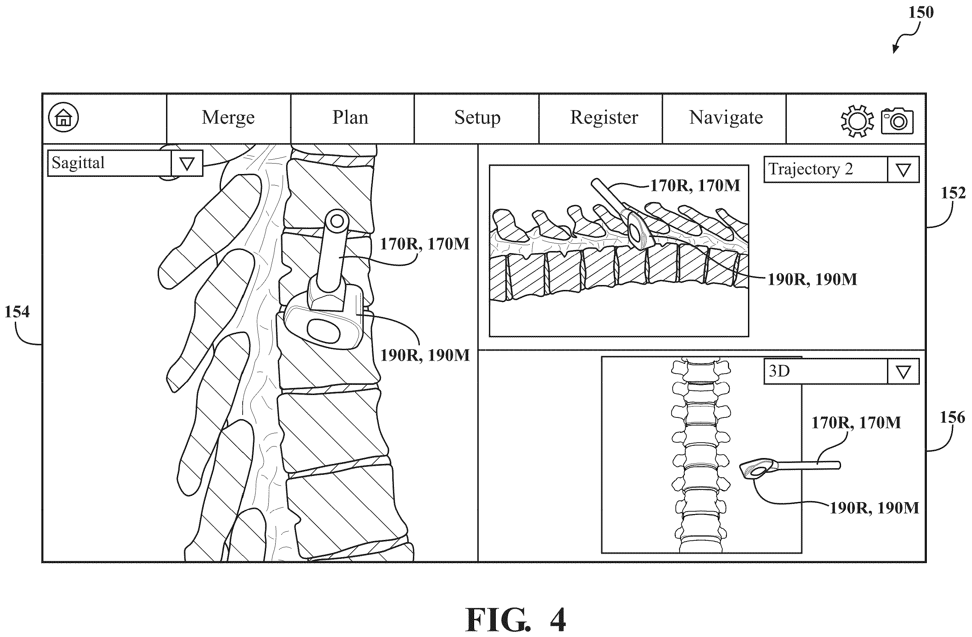

Referring to FIG. 4, an exemplary configuration of the graphic user interface (GUI) 150 of the surgical navigation system 110 is illustrated. The GUI 150 may be controlled by the controller 141 and configured as a touch screen on the display 120 of the surgical navigation system 110, an interactable object in a virtual/augmented reality space, or any suitable alternative.

In the illustrated implementation, the GUI 150 includes a first window 152, a second window 154, and a third window 156, each of which contain a view of a patient image. In this implementation, the first window 152 contains a trajectory view of the patient image, the second window 154 contains a sagittal view of the patient image, and the third window 156 contains a perspective view of a 3D model of the patient. The GUI 150 is shown operating in a navigation mode, but the GUI may also operate in other modes, such as a planning mode and a registration mode. The methods and functions of the system 100 are described with reference to the illustrated implementation in which the GUI 150 is operating in the navigation mode but may also be carried out while the GUI 150 is in the planning mode, the registration mode, or any other mode.

As described above, the display 120 is configured to display patient images (e.g., pre-operative patient images or intraoperative patient images) via the GUI 150. An entirety of the patient image, or a portion thereof, may be shown in the windows 152, 154, 156. The patient image may be a volumetric image, such as a CT image, and the windows 152, 154, 156 may each contain a "view" corresponding to the volumetric image. For example, each view may include at least a portion of a two-dimensional slice image derived from the volumetric image. Alternatively, the slice image may be a raw CT slice image upon which the volumetric image was generated. The views may alternatively include other two-dimensional images as long as a registration between the two-dimensional images and the volumetric images is known. In Figure 4, the views shown in the first and second windows 152, 154 each include a portion of a slice image which corresponds to slices of the volumetric image, and the view shown in the third window 156 includes a portion of a 3D model created based on the volumetric image. As such, the memory device may contain at least one slice image. The pose of the slice image(s) relative to the image coordinate system (and thus the volumetric image) may be known. The slice image(s) may be CT slice images and/or digitally reconstructed radiographs (DRRs).

A virtual representation of at least part of the surgical instrument 170 may be overlaid onto the patient image in at least one of the windows 152, 154, 156 based on the tracked pose of the surgical instrument 170 relative to the patient anatomy. Even further, a virtual representation the implant 190 may be overlaid onto the patient image in at least one of the windows 152, 154, 156 based on the pose of the implant 190 relative to the instrument 170. The pose of the implant 190 may be determined and/or updated according to the methods described below.

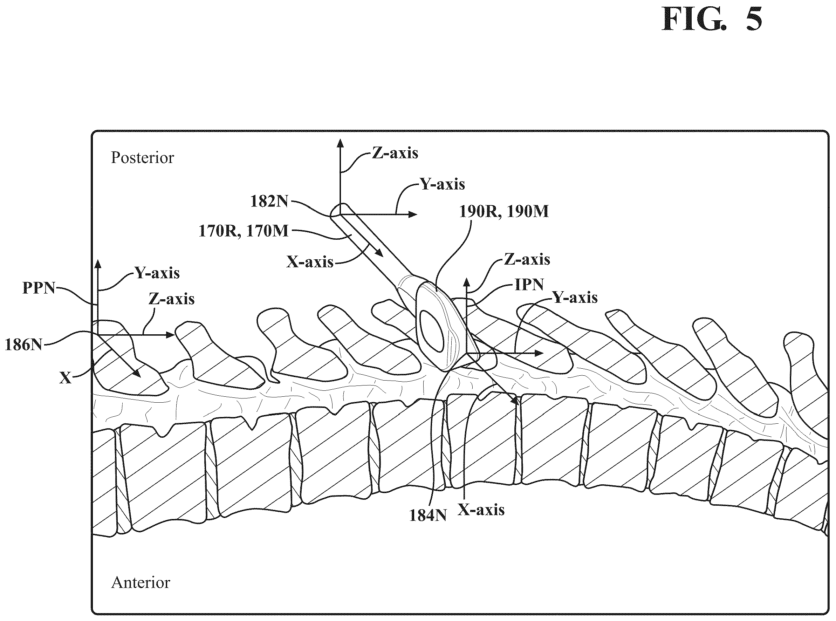

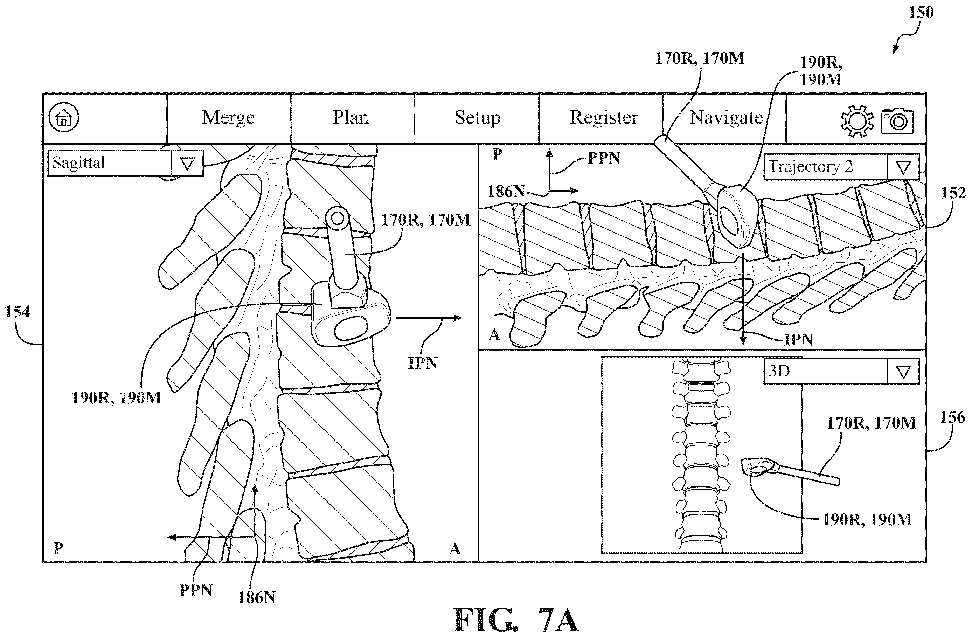

Referring to FIGS. 5-7B, a method 200 for displaying representations of the instrument 170 and the implant 190 relative to image data of the patient is illustrated. The method 200 is described as being carried out by the controller 141, but it is further contemplated to execute the method via at least one alternative computing device, such as a cloud computing device/environment. The controller 141 may also be used for part of the method 200, while the cloud computing device/environment is sued for the remainder of the method 200.

Starting with FIG. 5, representations of the instrument 170 and the implant 190 are shown overlaid onto image data of the patient. These representations are virtual representations generated and/or retrieved by the controller 141, and the representations include an instrument representation 170R and an implant representation 190R. The sizes and shapes of the representations 170R, 190R are based on a predetermined shape/size of the instrument 170 and the implant 190, respectively, both of which are known to the controller 141. For example, the controller 141 may have access to a database that includes a list of possible instruments and implants along with corresponding spatial dimensions. In some implementations, the instrument and implant representations 170R, 190R may include virtual models. For example, the instrument representation 170R may include an instrument model 170M, and the implant representation 190R may include an implant model 190M. The models 170M, 190M may be volumetric models designed to imitate the shape and size of their real-world counterparts 170, 190. As shown in FIG. 5, the instrument model 170M may include a set of instrument pose vectors 182N, and the implant model 190M may include a set of implant pose vectors 184N. Further, the image data may include a set of patient pose vectors 186N. Each set of pose vectors 182N, 184N, 186N may include corresponding sets of orthogonal X, Y, and Z vectors.

The instrument representation 170R may be overlaid onto the image data based on the tracked pose of the instrument 170. For example, the instrument model 170M may be overlaid onto the image data such that the instrument pose vectors 182N are oriented relative to the patient pose vectors 186N. The instrument and patient pose vectors 182N, 186N may be oriented based on the tracking data associated with the instrument tracker 180 and the patient tracker 181 to match/mimic the spatial relationship between the instrument 170 and the patient. A registration transform(s) may be used to relate the sets of pose vectors 182N, 184N, 186N to the image data. For example, an instrument registration transform may be combined with the tracked pose of the instrument tracker 180 to orient the instrument pose vectors 182N with the image data. Further, a patient registration transform may be combined with the tracked pose of the patient tracker 181 to orient the patient pose vectors 186N relative to the image data. Put simply, the instrument and patient pose vectors 182N, 186N represent the poses of the instrument coordinate system 182 and the patient coordinate system 186 relative to the localizer coordinate system 113.

Unlike the poses of the patient and the instrument representation 170R, the pose of the implant representation 190R may be based on a combination of the tracked pose of the instrument 170 and an expected spatial relationship between the patient and the implant 190. In the case of an asymmetrical spinal implant, like the implant 190 of the illustrated implementation, the implant 190 is often configured to be placed between adjacent vertebrae of the patient such that the thin side 195 of the implant 190 is facing towards a posterior side of the patient. As such, the system 100 may assume that the thin side 195 is facing towards the posterior side of the patient without needing to rely on tracking data specific to the implant 190. This is primarily because the system 100 may assume that the surgeon is introducing the implant 190 into the patient in the correct orientation. To this end, one vector of the set of implant pose vectors 184N may be automatically aligned with one vector of the set of patient pose vectors 186N so that the implant model 190M is in the expected orientation when overlaying the implant model 190M onto the image data of the patient. Herein, the Z-vector of the set of implant pose vectors 184N is referred to as an implant pose vector IPN, and the Y-vector of the set of patient pose vectors 186N is referred to as a patient pose vector PPN. When in the expected orientation, the implant pose vector IPN is oriented in a similar direction as the patient pose vector PPN, for example, oriented such that the implant pose vector IPN and the patient pose vector PPN form an angle below 90 degrees relative to the image data and/or localizer coordinate system 113. As mentioned above, this method avoids the need for calibrating the implant model using additional tools like digitizers, as well as removes the need for the user to provide input(s) to the controller 141 regarding the orientation of the implant 190 relative to the instrument 170.

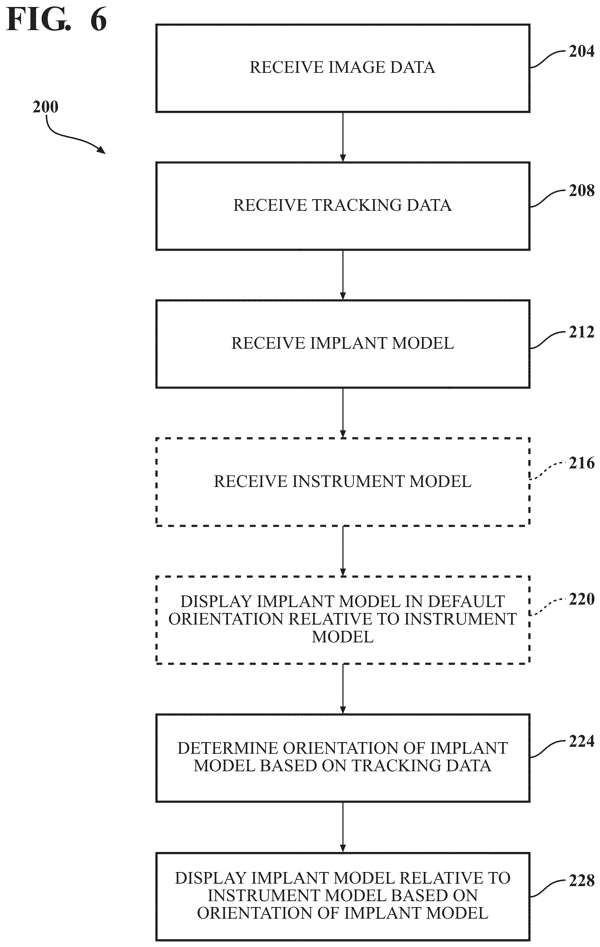

Moving to FIG. 6, a flowchart describing the method 200 for displaying representations of the instrument 170 and the implant 190 relative to image data of the patient is shown. As mentioned, the method 200 may be performed by any combination of capable computing device(s). Further, the controller 141 is described as receiving different types of data - this should be understood to include any means of making the data accessible to the controller 141, such as retrieving, receiving, accessing, and/or generating the data.

Starting at 204, image data representative of the patient is received or accessed by the controller 141. The image data may include intraoperative image data generated by the imaging system 160, or preoperative image data generated by the imaging system 160 or another imaging system. At 208, tracking data is received. The tracking data includes data indicative of a pose of the patient and data indicative of a pose of the instrument 170. For example, the tracking data may include a tracked pose of the instrument tracker 180 and a tracked pose of the patient tracker 181. In other implementations, the tracking data may be generated by machine vision techniques or suitable alternatives.

At 212, a model of the implant, such as the implant model 190M, is received by the controller 141. At 216, a model of the instrument, such as the instrument model 170M, may be received by the controller 141. The model may be selected from a set of models available to the controller 141 so as to match the instrument being navigated. At 220, the controller 141 may control the GUI 150 to display a representation of the implant 190 in a default orientation relative to a representation of the instrument 170. For example, the controller 141 may cause the GUI 150 to display the implant representation 190R relative to the instrument representation 170R such that the implant pose vector IPN is oriented in the same direction as the Z-vector of the set of instrument pose vectors 182N.

At 224, the controller 141 determines the orientation of the implant model, such as the orientation of the implant pose vector IPN of the implant model 190M, based on the tracking data and an expected relationship between the implant 190 and the patient. In other words, the controller 141 may determine how the representation of the implant should be oriented relative to the representation of the instrument, and this determination is based on the tracked pose of the instrument 170 relative to the tracked pose of the patient. As described above, the system 100 may assume that the implant 190 is being inserted into the patient in the correct orientation and determine the orientation of the implant as the correct orientation. In the illustrated implementation, this means that the implant model 190 is assumed to be oriented relative to the patient such that the implant pose vector IPN is oriented in a similar direction as the patient pose vector PPN for example, oriented such that the implant pose vector IPN and the patient pose vector PPN form an angle below 90 degrees relative to the image data and/or localizer coordinate system 113.

At 228, the controller 141 controls the GUI 150 to display a representation of the implant 190 relative to a representation of the instrument 170 based on the orientation of the implant model determined at 224. For example, the controller 141 may cause the GUI 150 to display the implant representation 190R relative to the instrument representation 170R such that the implant pose vector IPN is oriented in a similar direction as the patient pose vector PPN (*e.g.,* within 90 degrees of) and/or the same direction as the Z-vector of the set of instrument pose vectors 182N. If step 220 is carried out and the default orientation of the implant representation 190R is different from the orientation determined at 224, the implant representation 190R may be reoriented so that the implant pose vector IPN is oriented in a similar direction as the patient pose vector PPN, such as within 90 degrees thereof. Further, the method 200 may loop between steps 224 and 228 while continuously receiving tracking data. In effect, this allows the orientation of the implant representation 190R to be continuously updated as the instrument 170 and implant 190 are moved relative to the patient by the surgeon.

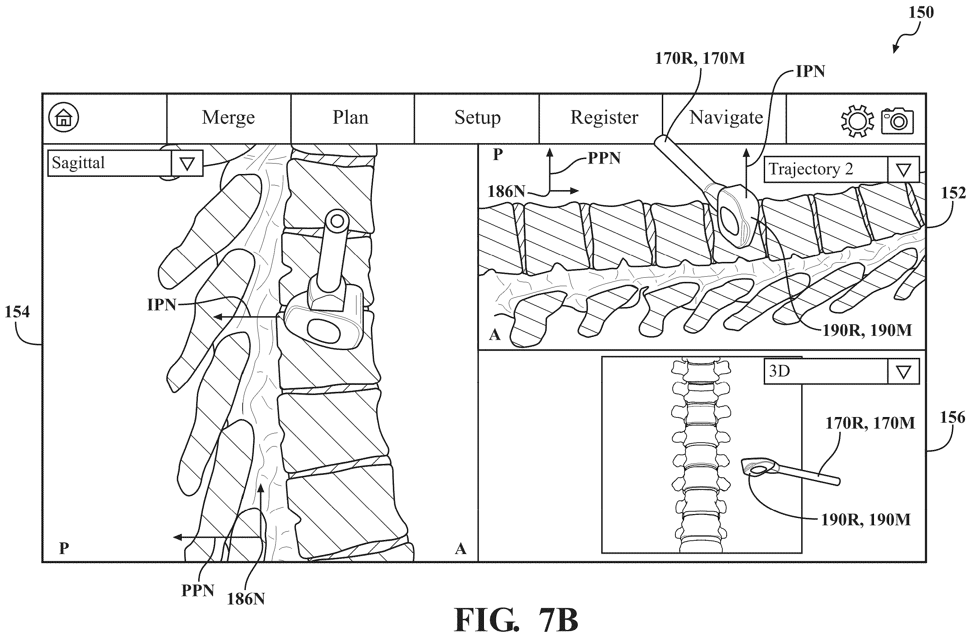

Referring to FIGS. 7A and 7B, the implant representation 190R is shown being reoriented relative to the instrument representation 170R according to the method 200 shown in FIG. 6. In FIG. 7A, the implant representation 190R is oriented such that the implant pose vector IPN is facing in a direction substantially opposite of (*e.g.,* between 91 and 269 degrees relative to) the patient pose vector PPN. Then, in FIG. 7B, the implant representation 190R has been reoriented such that the implant pose vector IPN is facing a similar direction as the patient pose vector PPN (e.g., within 90 degrees thereof). Reorientation of the implant representation 190R occurs at step 228 of the method 200 and may happen in response to the surgeon rotating the instrument 170 and/or the implant representation 190 being first displayed in the default orientation at step 220 which is later determined to be incorrect at step 224. In any case, the implant representation 190R is controlled by the controller 141 to match the expected orientation of the implant 190 relative to the patient (e.g., the thin side 195 facing the posterior side of the patient).

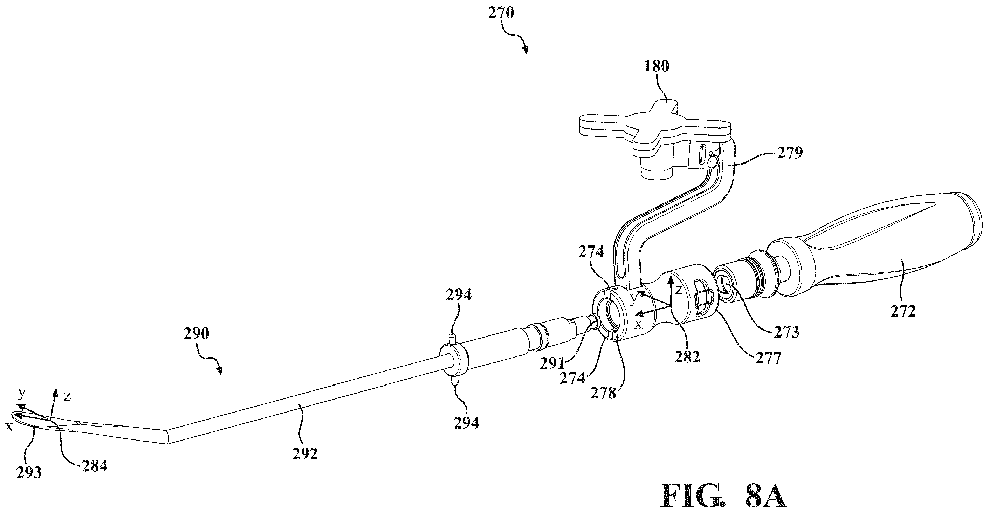

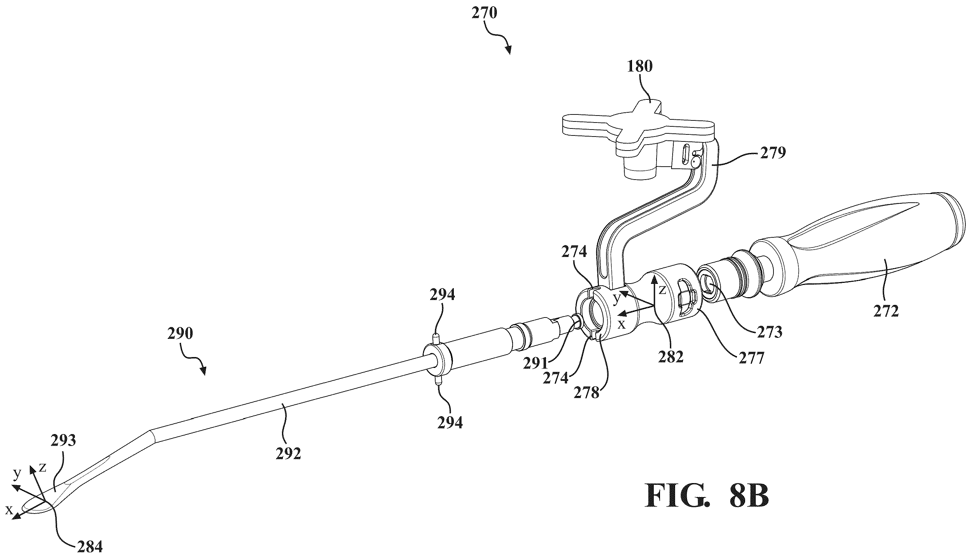

Referring to FIGS. 8A and 8B, a surgical instrument 270 according to another implementation is shown. In this implementation, the surgical instrument 270 is a spinal disc preparation instrument that is similar to the previously described instrument 170 in some respects. The instrument 270 of FIGS. 8A and 8B may include an instrument body 271 configured to receive a handle 272 and an end effector 290. The instrument body 271 includes a proximal end 277 for receiving the handle 272 and a distal end 278 for receiving the end effector 290. A tracking post 279 configured to receive the instrument tracker 180 may extend from the instrument body 271 such that the instrument body 271 acts as a tracker adapter for the instrument 270. More specifically, the handle 272 defines a channel 273 configured to receive a proximal end 291 of the end effector 290, and the channel 273 may receive the proximal end 281 of the end effector 290 without the instrument body 271 being present. By including the instrument body 271 between the handle 272 and end effector 290, however, the instrument tracker 180 may be coupled to the instrument 270 via the tracking post 279 and used to track a pose of the instrument 270.

The instrument 270 of FIGS. 8A and 8B is configured to receive the end effector 290. The end effector 290 may include the proximal end 291, a distal end 293, and a shaft 292 extending between the proximal and distal ends 291, 293. As previously mentioned, the proximal end 291 of the end effector 290 may be passed through/received by the instrument body 271 and removably coupled to the handle 272 via the channel 273 defined by the handle 272. Further, the end effector 290 may be asymmetrical about an axis of an instrument coordinate system 282. As will be appreciated from the figures, this may include a bend of the shaft 292 away from an x-axis of the instrument coordinate system 282. Even further, the end effector 290 may be configured to be coupled to the instrument body 271 in multiple configurations. For example, in the illustrated implementation, the instrument body 271 defines notches 274 configured to receive first and second alignment members 294A, 294B protruding from the shaft 292/proximal end 291 of the end effector 290. In FIG. 8A, the end effector 290 is shown in a first orientation in which the first alignment member 294A is received by the notch 274 arranged adjacent to the tracking post 279 such that the distal end 293 is bent upwards relative to the tracking post 279 *(i.e.,* in a positive z direction relative to the instrument coordinate system 282). Comparatively, in FIG. 8B, the end effector 290 is shown in a second orientation in which the second alignment member 294B is received by the notch 274 arranged adjacent to the tracking post 279 such that the distal end 293 is bent downwards relative to the tracking post 279 (*i.e.,* in a negative z direction relative to the instrument coordinate system 282).

During use, similar to the instrument 170 of the previous implementation, the navigation system 110 may be used to track the pose of the instrument 270, and the end effector 290 coupled thereto, by tracking the tracking device 180 coupled to the instrument 270. As briefly mentioned above, the navigation system 110 may associate the instrument coordinate system 282 with the instrument 270, the instrument coordinate system 282 including corresponding X, Y, and Z axes. Additionally, the navigation system 110 may associate an end effector coordinate system 284 with the end effector 290, the end effector coordinate system 284 including X, Y, and Z axes. The navigation system 110 may then track the poses of the instrument and end effector coordinate systems 282, 284 relative to the localizer coordinate system 113 by tracking the instrument tracker 180.

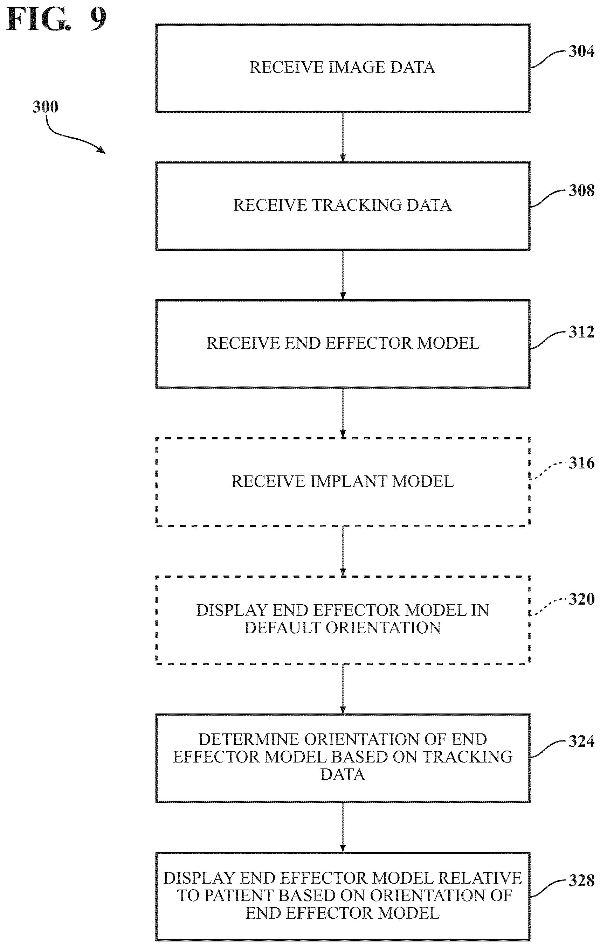

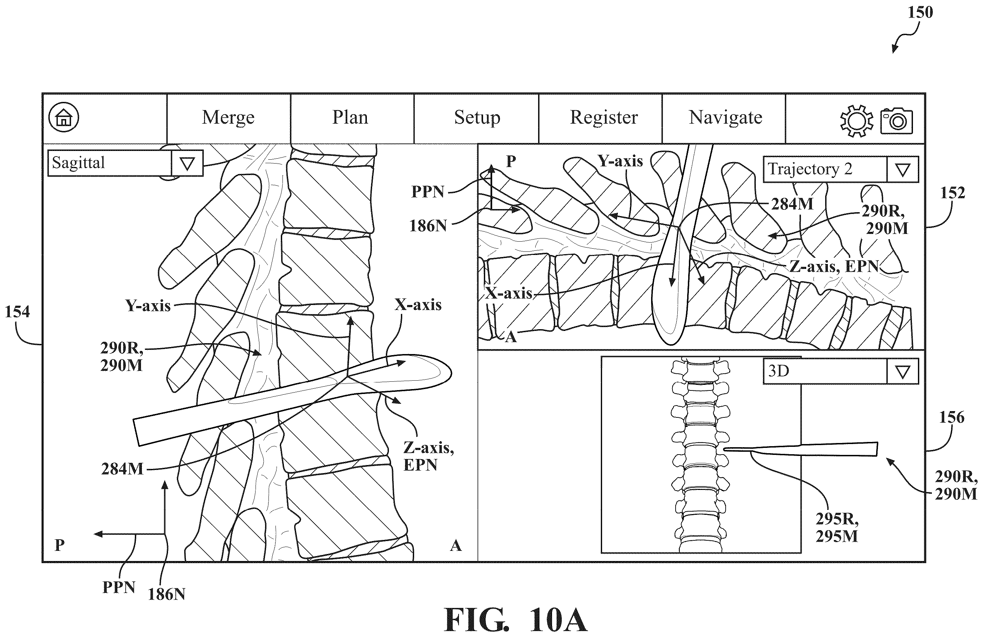

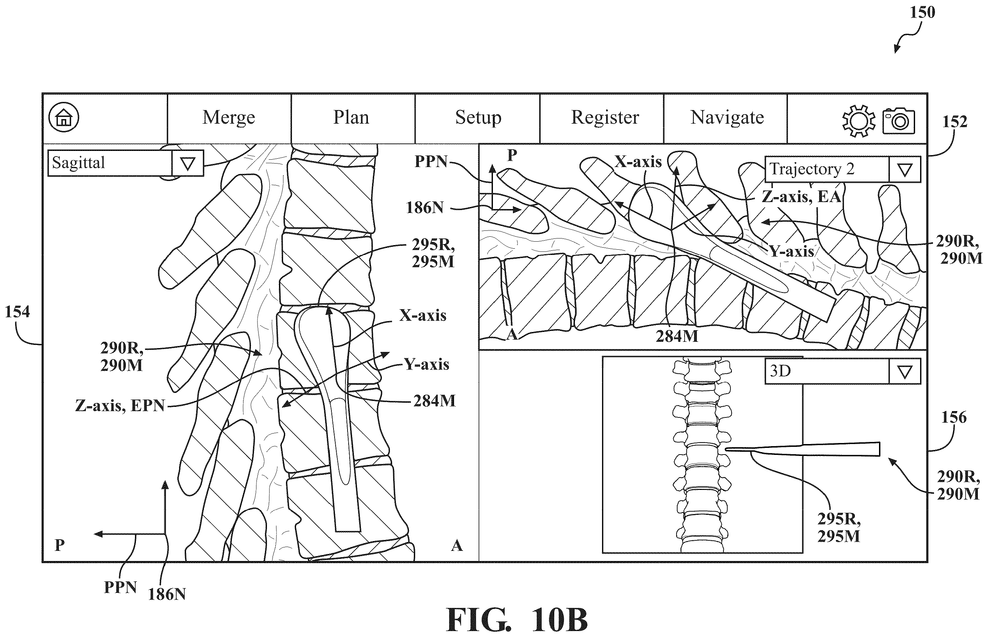

Referring to FIGS. 9-10B, a method 300 for displaying representations of the instrument 270 and/or the end effector 290 relative to image data of the patient is illustrated. Similar to the asymmetrical implant 190 of the previous implementation, the system 100 may assume that the asymmetric end effector 290 is in one of the first and second orientation based on a combination of the tracked pose of the instrument 270 and an expected spatial relationship between the patient and the end effector 290. To this end, a flowchart depicting the method 300 is shown in FIG. 9, and reorientation of the end effector 290 is illustrated in FIGS. 10A and 10B. The method 300 is described as being carried out by the controller 141, but the method 300 may be performed by any combination of capable computing device(s). Further, the controller 141 is described as receiving different types of data - this should be understood to include any means of making the data accessible to the controller 141, such as retrieving, receiving, accessing, and/or generating the data.

Starting at 304, image data representative of the patient is received or accessed by the controller 141. The image data may include intraoperative image data generated by the imaging system 160, or preoperative image data generated by the imaging system 160 or another imaging system. At 308, tracking data is received. The tracking data includes data indicative of a pose of the patient and data indicative of a pose of the instrument 270. For example, the tracking data may include a tracked pose of the instrument tracker 180 coupled to the instrument 270 and a tracked pose of the patient tracker 181. In other implementations, the tracking data may be generated by machine vision techniques or suitable alternatives.

At 312, a model of the end effector, such as an end effector model 290M (shown in FIGS. 10A and 10B), is received by the controller 141. In some implementations, at 316, a model of an implant may also be received by the controller 141. The instrument model 290M, and optionally the implant model, may be selected from a set of models available to the controller 141 so as to match the end effector and/or implant being navigated. In some implementations, the end effector 290 may include a trial implant integrally formed with the end effector 290. In these cases, the end effector model 290M may include the implant model. Like the instrument and implant models 170M, 190M described above, the end effector model 290M may include a set of end effector pose vectors 282V and the implant model may include a set of implant pose vectors, each including corresponding sets of orthogonal X, Y, and Z vectors.

At 320, the controller 141 may control the GUI 150 to display a representation of the end effector 290 in a default orientation relative to the tracking data. In other words, the tracking data may include the pose of the instrument tracker 180 relative to the pose of the patient tracker 181, and the representation of the end effector 290R may be overlaid onto the image data such that the pose of the end effector coordinate system 284 is oriented relative to the tracking device 180 like shown in FIG. 8A. For example, the controller 141 may cause the GUI 150 to display an end effector representation 290R relative to the set of patient pose vectors 186N as if the end effector 290 was coupled to the instrument body 271 in the first orientation shown in FIG. 8A.

At 324, the controller 141 determines the orientation of an end effector pose vector associated with the end effector model 290M, such as an end effector pose vector EPN (shown in FIGS. 10A and 10B), based on the tracking data and an expected relationship between the end effector 290 and the patient. Said differently, the controller 141 determines how the representation of the end effector 290R should be oriented relative to the representation of the patient, and this determination is based on the tracked pose of the instrument 270 relative to the tracked pose of the patient. As described above, the system 100 may assume that the end effector 290 is being inserted into the patient in the correct orientation and determine the orientation of the end effector as the correct orientation. In the illustrated implementation, this means that the end effector model 290M is assumed to be oriented such that the end effector pose vector EPN is oriented in a similar direction as the patient pose vector PPN, such as forming an angle within 90 degrees relative to the image data therewith.

At 328, the controller 141 controls the GUI 150 to display a representation of the end effector 290 relative to the representation of the patient based on the orientation of the end effector pose vector EPN determined at 324. For example, the controller 141 may cause the GUI 150 to display the end effector representation 290R relative to the image data such that the end effector pose vector EPN is oriented in a similar direction as (*e.g.,* within 90 degrees relative to) the patient pose vector PPN. If step 320 is carried out and the default orientation of the end effector representation 290R is different from the orientation determined at 324, the end effector representation 290R may be reoriented so that the end effector pose vector EPN is oriented in a similar direction as the patient pose vector PPN. Further, the method 300 may loop between steps 324 and 328 while continuously receiving tracking data. In effect, this allows the orientation of the end effector representation 290R to be continuously updated as the instrument 270 and the end effector 290 are moved relative to the patient by the surgeon.

Referring to FIGS. 10A and 10B, the end effector representation 290R is shown being reoriented relative to the image data according to the method 300 shown in FIG. 9. In FIG. 10A, the end effector representation 290R is oriented such that the end effector pose vector EPN is facing the in a direction substantially opposite of the patient pose vector PPN (*e.g.,* such that the vectors EPN, PPN form an angle between 91 and 269 degrees). Then, in FIG. 10B, the end effector representation 290R has been reoriented such that the end effector pose vector EPN is facing a direction similar to the patient pose vector PPN. Reorientation of the end effector representation 290R occurs at step 328 of the method 300 and may happen in response to the surgeon rotating the instrument 270 and/or the end effector representation 290R being first displayed in the default orientation at step 320 which is later determined to be incorrect at step 324. In any case, the end effector representation 290R is controlled by the controller 141 to match the expected orientation of the end effector 290 relative to the patient (*e.g.,* angled towards the posterior side of the patient).

Similar to the prior method 200, the present method 300 avoids the need for the user to provide input(s) to the controller 141 regarding the configuration of the end effector 290 relative to the instrument 270, as well as avoids requiring additional sensors in the instrument 270 that are configured to sense the configuration of the end effector.

In the above description of the methods 200, 300, the implant and end effector representations 190R, 290R are described as being reoriented based on relationships between the patient pose vector PPN and the implant pose vector IPN/end effector pose vector EPN. In effect, this allows the system 100 to "flip" the implant representation 190R and/or the end effector representation 290R relative to the image data and/or instrument representation 170R so as to match the expected/correct orientation of the implant 190 and/or end effector 290 relative to the patient and/or the instrument 170, 270. In some cases, this may be more specifically implemented. For example, the controller 141 may define a misalignment threshold meant to represent a maximum misalignment between (1) the displayed representation of the implant 190 and/or end effector 290 and (2) the expected/correct representation of the implant 190 and/or end effector 290. Then, the controller 141 may determine an alignment metric, such as at step 224 and/or step 324, and reorient the implant 190 and/or end effector 290 if the alignment metric is higher than the misalignment threshold. In either method 200, 300, the misalignment threshold may be defined as a specific angle, such as 90 degrees.

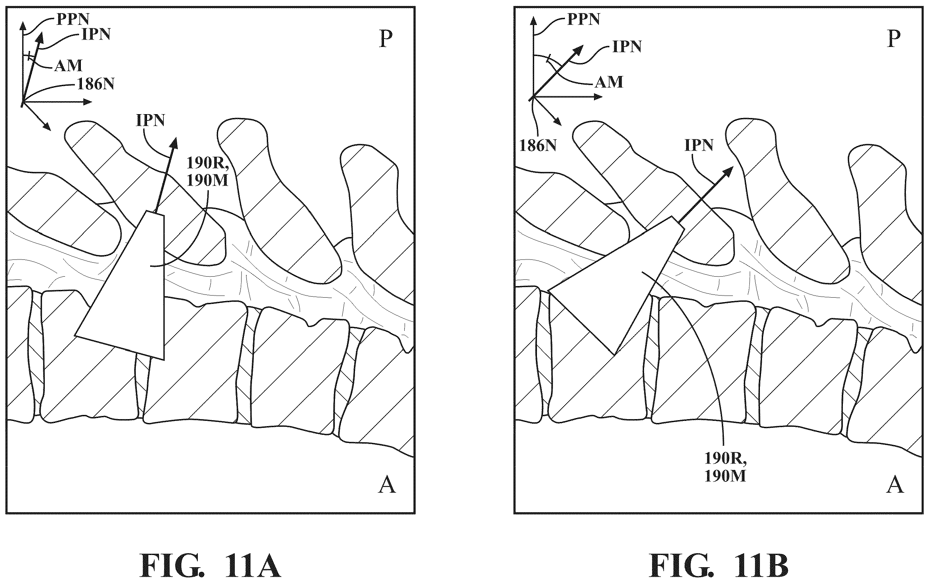

Referring to FIGS. 11A-11D, an implementation of reorienting the implant representation 190R relative to the image data in line with the above example is shown. In each of these figures, the implant representation 190R (*e.g.,* the implant model 190M) is shown overlaid onto image data of the patient, and the set of patient pose vectors 186N is shown. In this implementation, an alignment metric AM may be calculated as an angle formed by the implant pose vector IPN and the patient pose vector PPN. The implant representation 190R may be flipped (e.g., rotated 180 degrees about the z-vector of the instrument representation and/or the x-vector of the implant representation) if the angle formed by the implant pose vector IPN and the patient pose vector PPN meets or exceeds the misalignment threshold. The misalignment threshold is equal to 90 degrees in this implementation but may be set to other values depending on the needs of the surgeon and/or the shape of the implant 190 being navigated. Further, although the instrument representation 170R is omitted from these figures, reorienting the implant representation 190R should be understood to optionally include rotating the implant representation 190R relative to the instrument representation 170R as described above.

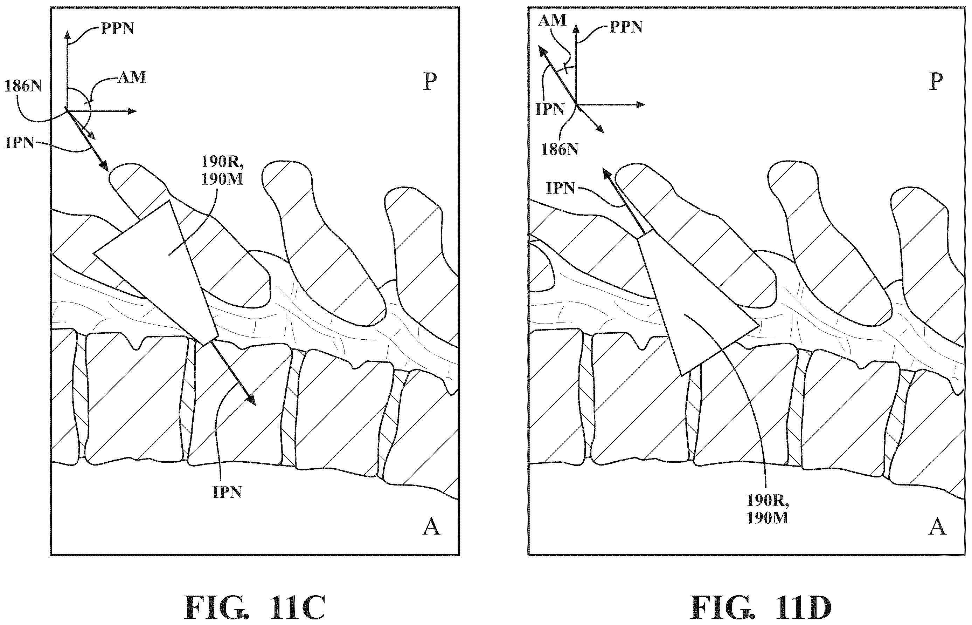

Starting with FIG. 11A, the implant representation 190R is slightly rotated relative to the patient pose vector PPN, but the alignment metric AM is shown to be less than the misalignment threshold. Moving from FIG. 11A to 11B, the instrument and implant 170, 190 have been rotated relative to the patient and the alignment metric AM has increased relative to FIG. 11A. Since the alignment metric AM is still less than the misalignment threshold in this example, the implant representation 190R is not reoriented relative to the instrument representation or the patient. Looking to FIG. 11C, the instrument and implant 170, 190 have been further rotated relative to the patient and the alignment metric AM has increased relative to FIGS. 11A and 11B. At this point, the alignment metric AM is greater than the misalignment threshold, which is equal to 90 degrees in this example. Thus, the implant representation 190R is reoriented as shown in FIG. 11D. Depending on the implementation, the implant representation 190R may be rotated 180 degrees about the x-axis of the implant coordinate system 184 and/or the x-axis of the instrument coordinate system 182 (see FIGS. 3A and 3B). The calculation of the alignment metric AM, and the comparison of the metric AM to the misalignment threshold may occur during steps 224 and 228 of the method 200 depicted in FIG. 6.

In some implementations, the implant model 190M may be represented in either of a first orientation or a second orientation relative to the z-vector of the model instrument 170M, such as the orientations shown in FIGS. 7A and 7B, and the controller 141 may select one of the orientations based on the misalignment threshold and the alignment metric. For example, the first orientation of the implant model 190R may be the default orientation described in reference to step 220 of the method 200 of FIG. 6, and the controller 141 may display the implant representation 190R in the second orientation relative to the instrument representation 170R if the alignment metric meets or exceeds the misalignment threshold.

Additionally, or alternatively, although FIGS. 11A-11D and the corresponding description above are focused on reorienting the asymmetric implant representation 190R relative to the image data, a similar process may be carried out for asymmetric end effectors, such as the end effector 290 of the surgical instrument 270. For the method 300 of FIG. 9, the alignment metric may be calculated as an angle formed by the end effector pose vector EPN and the patient pose vector PPN. Here, the end effector representation 290R may be flipped (*e.g.,* rotated 180 degrees about the y-vector of the end effector representation 290R) if the angle formed by the implant pose vector IPN and the patient pose vector PPN meets or exceeds 90 degrees. Further, in some implementations, the end effector model 290M may be represented in either of a first orientation or a second orientation, such as the configurations shown in FIGS. 10A and 10B, and the controller 141 may select one of the orientations based on the misalignment threshold and the alignment metric. For example, the first orientation of the end effector model 290R may be the default orientation described in reference to step 320 of the method 300 of FIG. 9, and the controller 141 may display the end effector representation 290R in the second orientation relative to the tracking data if the alignment metric meets or exceeds the misalignment threshold.

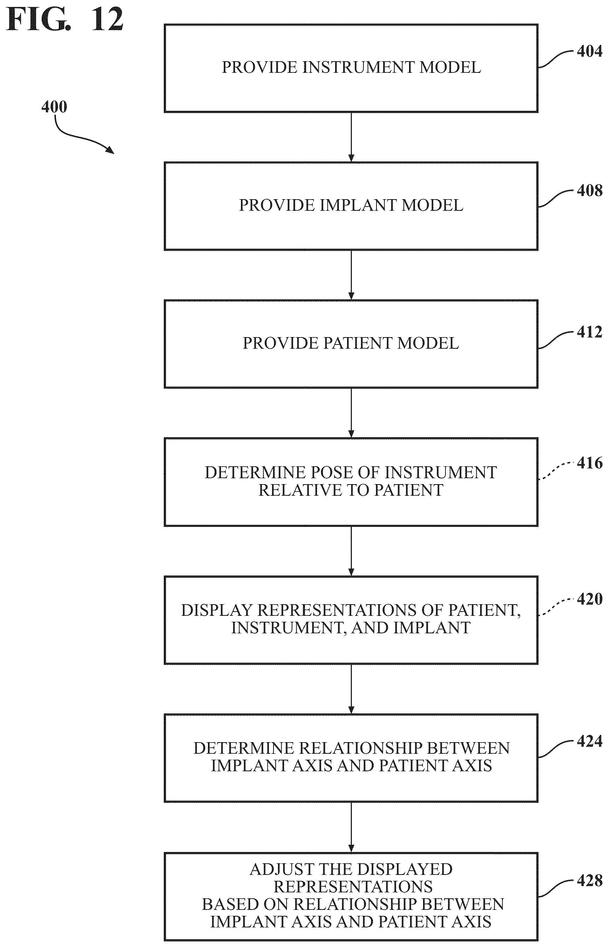

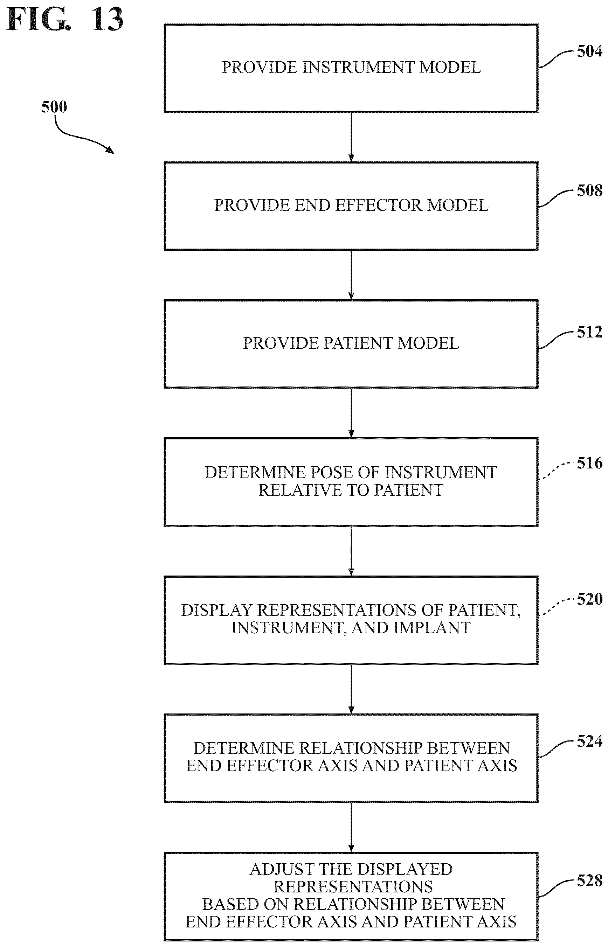

Referring to FIGS. 12 and 13, additional implementations of the methods described in reference to FIGS. 6 and 9 are shown. These implementations are described as being carried out by the controller 141 but may be performed by any capable computing device(s). Further, it is contemplated to combine any or all of these implementations with any or all of the previous implementations. Starting with FIG. 12, a flowchart describing a method 400 for displaying representations of the instrument 170 and the implant 190 relative to a representation of the patient is shown.

At 404, an instrument model is provided to the controller 141. This step may include determining the identity of the instrument, and then providing an instrument model associated with the identified instrument. The instrument model may be like that shown in FIGS. 7A and 7B (*i.e.,* similar to the instrument model 170M) and may include an x-vector and a z-vector. At 408, an implant model is provided to the controller 141. Similar to step 404, step 408 may include determining the identity of the implant, and an implant model associated with the identified implant may be provided. The implant model may be like that shown in FIGS. 7A and 7B (*i.e.,* similar to the implant model 190M) and may include an implant pose vector. At 412, a patient model is provided to the controller 141. The patient model may be a volumetric model of at least a portion of the patient (*e.g.,* the spine of the patient), such as a model generated by segmenting volumetric CT image data. After the instrument, implant, and patient models have been provided, the method continues to step 416.

At 416, the tracked pose of the instrument relative to the tracked pose of the patient is determined by the controller 141. Then, at 420, the controller 141 displays the patient representation, the instrument representation, and the implant representation relative to one another. In one example, step 416 may include determining the pose of the instrument tracker 180 and the pose of the patient tracker 181. In this example, step 420 may include causing the GUI 150 to display the instrument representation 170R relative to the patient representation (*e.g.,* like shown in the third window 156 illustrated in FIG. 7A) and causing the GUI 150 to display the implant representation 190R in a first/default orientation relative to the instrument representation 170R. At 424, the controller 141 determines the relationship between the implant pose vector IPN and the patient pose vector PPN. This may be performed similar to step 224 of the method 200 shown in FIG. 6. At 428, the controller 141 adjusts the pose of the implant representation relative to the pose of the instrument representation based on the relationship between the implant pose vector IPN and the patient pose vector PPN as determined at 420. This may be performed like step 228 and/or include a calculation of an alignment metric, and a comparison of the alignment metric to a misalignment threshold, like described in reference to FIGS. 11A-11D.

Now looking to FIG. 13, a method 500 for displaying representations of the instrument 270 and the end effector 290 relative to a representation of the patient is illustrated. At 504, an instrument model is provided to the controller 141. Similar to step 404 of the previous method 400, step 504 may include determining the identity of the instrument, and then providing an instrument model associated with the identified instrument. The instrument model may be representative of the instrument 270 shown in FIGS. 8A and 8B and may include an x-vector and a z-vector. At 508, an end effector model may be provided to the controller 141. Similar to step 408, step 508 may include determining the identity of the end effector, and an end effector model associated with the identified end effector may be provided. The end effector model may be like that shown in FIGS. 10A and 10B (*i.e.,* similar to the end effector model 290M) and may include an end effector pose vector. At 512, a patient model is provided to the controller 141. The patient model may be a volumetric model of at least a portion of the patient (*e.g.,* the spine of the patient), such as a model generated by segmenting volumetric CT image data and may include a patient pose vector. After the instrument, end effector, and patient models have been provided, the method continues to step 516.

At 516, the tracked pose of the instrument relative to the tracked pose of the patient may be determined by the controller 141. Then, at 520, the controller 141 may display the patient representation, the instrument representation, and the end effector representation relative to one another. Depending on the implementation, the instrument representation may be omitted and not displayed similar to the implementation of FIGS. 10A and 10B. In an example, step 516 may include determining the pose of the instrument tracker 180 and the pose of the patient tracker 181. Continuing the example, step 520 may include causing the GUI 150 to display the end effector representation 290R relative to the patient representation based on the tracked pose of the instrument 270 and as if the end effector 290 was coupled to the instrument 270 in a first/default orientation. At 524, the controller 141 determines the relationship between the end effector pose vector EPN and the patient pose vector PPN. This may be performed similar to step 324 of the method 300 shown in FIG. 9. At 528, the controller 141 adjusts the pose of the end effector representation relative to the pose of the patient representation based on the relationship between the end effector pose vector and the patient pose vector as determined at 520. This may be performed like step 328 and/or include a calculation of an alignment metric, and a comparison of the alignment metric to a misalignment threshold, like described in reference to FIGS. 11A-11D.

Although it is not described in detail above, it is further contemplated to combine at least a portion of the methods 200, 400 focused on implants with at least a portion of the methods 300, 500 focused on end effectors. For example, in the case of an asymmetrical end effector configured to receive an asymmetrical implant and be removably coupled to an instrument. In such cases, the system 100 may reorient at least one of the end effector and the implant coupled thereto based on an orientation of the end effector and/or implant relative to the patient. In one implementation, the controller 141 may compare both of the implant pose vector IPN and the end effector pose vector EPN to the patient pose vector PPN and reorient either of the end effector and implant in accordance with the methods described herein.

Several embodiments have been discussed in the foregoing description. However, the implementations discussed herein are not intended to be exhaustive or limit the filter assembly to any particular form factor. The terminology which has been used is intended to be in the nature of words of description rather than of limitation. Many modifications and variations are possible in light of the above teachings and the system may be practiced otherwise than as specifically described.

Certain implementations may be described with reference to the following clauses:
A1. A computer-program product or computer-implemented method for displaying an asymmetrical implant relative to image data representative of a patient, wherein the asymmetrical implant is configured to be attached to an implant insertion instrument in one of multiple orientations, the computer-program product or computer-implemented method: providing an instrument model associated with the implant insertion instrument, the instrument model including an x-vector and a z-vector; providing an implant model associated with the asymmetrical implant, the implant model including an implant pose vector; providing a patient model associated with the patient, the patient model including a patient pose vector; determining a pose of the implant insertion instrument relative to the patient; displaying: a representation of the patient, a representation of the instrument model, and a representation of the implant model oriented such that the implant pose vector is in a first orientation relative to the x and z axes of the instrument model; determining an expected relationship between the implant pose vector and the patient pose vector of the patient model; and adjusting the displayed representation of the implant model relative to the instrument model such that the implant pose vector in is a second orientation relative to the x and z axes based on the expected relationship between the implant pose vector and the patient pose vector of the patient model.
A2.The computer-program product or computer-implemented method of clause A1, wherein the implant pose vector is oriented in the same direction as the z-vector of the instrument model in the first orientation.
A3.The computer-program product or computer-implemented method of clause A2, wherein: the x-vector of the instrument model is perpendicular to the z-vector of the instrument model; and the second orientation of the implant pose vector is rotated about the x-vector relative to the first orientation of the implant pose vector.
A4. The computer-program product or computer-implemented method of clause A3, wherein the implant pose vector is oriented opposite of the z-vector of the instrument model in the second orientation.
A5.The computer-program product or computer-implemented method of any preceding clause, wherein the relationship between the implant pose vector and the patient pose vector is an alignment metric equal to an angle formed by the implant pose vector and the patient pose vector.
A6.The computer-program product or computer-implemented method of clause A5, further comprising: defining a misalignment threshold; determining the alignment metric; selecting one of the first orientation of the implant pose vector and the second orientation of the implant pose vector based on the misalignment threshold and the alignment metric; and adjusting the displayed representation of the implant model relative to the instrument model in response to the selected orientation being different than the displayed orientation.
A7.The computer-program product or computer-implemented method of any preceding clause, wherein: the representation of the instrument model is displayed relative to the representation of the patient model based on the pose of the implant insertion instrument relative to the patient, and the representation of the implant model is displayed relative to the representation of the instrument model.
A8. The computer-program product or computer-implemented method of any preceding clause, wherein the implant pose vector points from one side of the model of the asymmetrical implant to an opposing side of the model of the asymmetrical implant.
A9. The computer-program product or computer-implemented method of any preceding clause, wherein: the asymmetrical implant is a cage implant with a wedge-shaped body including a thick side and an opposing thin side; the model of the asymmetrical implant comprises a cage implant model having a thick side and an opposing thin side; and the implant pose vector points from the thick side toward the thin side of the cage implant model.
A10. The computer-program product or computer-implemented method of any preceding clause, comprising: to determine a patient pose vector from the image data representative of the patient, the patient pose vector having an orientation that points toward a certain anatomical direction of the patient in a coordinate system of the patient represented by the image data.
A11. The computer-program product or computer-implemented method of clause A10, comprising: to determine the orientation of the implant pose vector relative to the coordinate system of the patient represented by the image data; detect that the implant pose vector and the patient pose vector are oppositely directed; and in response to detecting that the implant pose vector and the patient pose vector are oppositely directed, automatically reorient the representation of the model of the asymmetrical implant to align the orientation of the implant pose vector with the orientation of the patient pose vector.
A12. The computer program product of clause A11, wherein the patient pose vector is a posterior vector that has an orientation that points toward a posterior side of the patient in the coordinate system of the patient.
B1. A computer-program product or computer-implemented method for displaying an asymmetrical end effector relative to image data representative of a patient, wherein the asymmetrical end effector is configured to be attached to an instrument in one of multiple orientations, the computer-program product or computer-implemented method: receiving the image data representative of the patient; receiving tracking data indicative of a pose of the patient and a pose of the instrument; receiving a model of the asymmetrical end effector which includes an end effector pose vector; determining an orientation of the end effector pose vector of the model based on the tracking data and an expected relationship between the asymmetrical end effector and the patient; and displaying a representation of the model of the asymmetrical end effector relative to a representation of the instrument based on the determined orientation of the end effector pose vector.
B2. The computer-program product or computer-implemented method of clause B1, further comprising displaying the representation of the instrument relative to a representation of the patient based on the tracking data.
B3. The computer-program product or computer-implemented method of clause B1, further comprising: defining a patient pose vector relative to the image data based on the tracking data; and determining the orientation of the end effector pose vector of the model based on an expected relationship between the end effector pose vector and the patient pose vector.
B4. The computer-program product or computer-implemented method of clause B3, wherein the patient pose vector is defined relative to the image data based on the tracking data and a patient registration transform.
B5. The computer-program product or computer-implemented method of clause B3, wherein determining the orientation of the end effector pose vector of the model includes aligning the end effector pose vector relative to the patient pose vector.
B6. The computer-program product or computer-implemented method of clause B1, wherein the orientation of the end effector pose vector is realized as a first orientation of the end effector pose vector relative to a z-vector of the representation of the instrument, and the method further comprises displaying the representation of the model of the asymmetrical end effector in the first orientation relative to the representation of the instrument as the instrument is moved relative to the patient.
B7. The computer-program product or computer-implemented method of clause B6, further comprising: determining an alignment metric based on the end effector pose vector and a patient pose vector as the instrument is moved relative to the patient; determining a second orientation of the end effector pose vector of the model relative to the z-vector of the representation of the instrument which is different from the first orientation; and displaying the representation of the model of the asymmetrical end effector in the second orientation relative to the representation of the instrument based on the alignment metric.
B8. The computer-program product or computer-implemented method of clause B7, wherein: the alignment metric is an angle formed by the end effector pose vector and the patient pose vector; and comparing the alignment metric to a misalignment threshold; selecting one of the first orientation and the second orientation based on the comparison, and displaying the representation of the model of the asymmetrical end effector in the selected one of the first orientation and the second orientation.
B9. The computer-program product or computer-implemented method of clause B7, wherein the second orientation of the end effector pose vector is rotated about an x-vector of the representation of the instrument relative to the first orientation of the end effector pose vector.
B 10. The computer-program product or computer-implemented method of any preceding clause, wherein the end effector pose vector points from one side of the model of the asymmetrical end effector to an opposing side of the model of the asymmetrical end effector.
B11. The computer-program product or computer-implemented method of any preceding clause, comprising: to determine a patient pose vector from the image data representative of the patient, the patient pose vector having an orientation that points toward a certain anatomical direction of the patient in a coordinate system of the patient represented by the image data.
B12. The computer-program product or computer-implemented method of any preceding clause, comprising to: determine the orientation of the end effector pose vector relative to the coordinate system of the patient represented by the image data; detect that the end effector pose vector and the patient pose vector are oppositely directed; and in response to detecting that the end effector pose vector and the patient pose vector are oppositely directed, automatically reorient the representation of the model of the asymmetrical end effector to align the orientation of the end effector pose vector with the orientation of the patient pose vector.
C1. A computer-program product or computer-implemented method for displaying an asymmetrical end effector relative to image data representative of a patient, wherein the asymmetrical end effector is configured to be attached to an instrument in one of multiple orientations, the computer-program product or computer-implemented method: providing an instrument model associated with the instrument, the instrument model including an x-vector and a z-vector; providing an end effector model associated with the asymmetrical end effector, the end effector model including an end effector pose vector; providing a patient model associated with the patient, the patient model includes a patient pose vector; determining a pose of the instrument relative to the patient; displaying: a representation of the patient, a representation of the instrument model, and a representation of the end effector model oriented such that the end effector pose vector is in a first orientation relative to the x and z axes of the instrument model; determining an expected relationship between the end effector pose vector and the patient pose vector of the patient model; and adjusting the displayed representation of the end effector model relative to the instrument model such that the end effector pose vector in is a second orientation relative to the x and z axes based on the expected relationship between the end effector pose vector and the patient pose vector of the patient model.
C2.The computer-program product or computer-implemented method of clause C1, wherein the end effector pose vector is oriented in the same direction as the z-vector of the instrument model in the first orientation.
C3. The computer-program product or computer-implemented method of clause C2, wherein: the x-vector of the instrument model is perpendicular to the z-vector of the instrument model; and the second orientation of the end effector pose vector is rotated about the x-vector relative to the first orientation of the end effector pose vector.
C4. The computer-program product or computer-implemented method of clause C3, wherein the end effector pose vector is oriented opposite of the z-vector of the instrument model in the second orientation.
C5. The computer-program product or computer-implemented method of clause C1, wherein the relationship between the end effector pose vector and the patient pose vector is an alignment metric equal to an angle formed by the end effector pose vector and the patient pose vector.
C6. The computer-program product or computer-implemented method of clause C5, further comprising: comparing the alignment metric to a misalignment threshold, selecting one of the first orientation of the end effector pose vector and the second orientation of the end effector pose vector based on the comparison; adjusting the displayed representation of the end effector model relative to the instrument model in response to the selected orientation being different than the displayed orientation.
C7. The computer-program product or computer-implemented method of clause C1, wherein: the representation of the instrument model is displayed relative to the representation of the patient model based on the pose of the instrument relative to the patient, and the representation of the end effector model is displayed relative to the representation of the instrument model.
C8. The computer-program product or computer-implemented method of any preceding clause, wherein the end effector pose vector points from one side of the model of the asymmetrical end effector to an opposing side of the model of the asymmetrical end effector.
C9. The computer-program product or computer-implemented method of any preceding clause, comprising: to determine a patient pose vector from the image data representative of the patient, the patient pose vector having an orientation that points toward a certain anatomical direction of the patient in a coordinate system of the patient represented by the image data.
C10. The computer-program product or computer-implemented method of any preceding clause, comprising to: determine the orientation of the end effector pose vector relative to the coordinate system of the patient represented by the image data; detect that the end effector pose vector and the patient pose vector are oppositely directed; and in response to detecting that the end effector pose vector and the patient pose vector are oppositely directed, automatically reorient the representation of the model of the asymmetrical end effector to align the orientation of the end effector pose vector with the orientation of the patient pose vector.

## Claims

1. A computer program product for use with a surgical system (100) that includes an implant insertion instrument (170) configured to attach to an asymmetrical implant (190) in one of multiple orientations, a localizer (112) configured to track the implant insertion instrument (170) and a patient, and a display device (120), wherein the computer program product comprises instructions, which are executable by one or more processors, to:
receive image data representative of the patient;
receive, from the localizer (112), tracking data indicative of a pose of the patient and a pose of the implant insertion instrument (170);
receive a model (190M, 190R) of the asymmetrical implant (190) which includes an implant pose vector (IPN);
determine an orientation of the implant pose vector (IPN) relative to the image data based on the tracking data; and
present, on the display device (120), a representation of the model (190M, 190R) of the asymmetrical implant (190) relative to a representation (170M, 170R) of the implant insertion instrument (170) based on the determined orientation of the implant pose vector (IPN).

2. The computer program product of claim 1, wherein the implant pose vector (IPN) points from one side of the model (190M, 190R) of the asymmetrical implant (190) to an opposing side of the model (190M, 190R) of the asymmetrical implant (190).

3. The computer program product of any preceding claim, wherein:
the asymmetrical implant (190) is a cage implant with a wedge-shaped body including a thick side (196) and an opposing thin side (195);
the model (190M, 190R) of the asymmetrical implant (190) comprises a cage implant model (190M, 190R) having a thick side and an opposing thin side; and
the implant pose vector (IPN) points from the thick side toward the thin side of the cage implant model (190M, 190R).

4. The computer program product of any preceding claim, wherein the instructions are executable by the one or more processors to determine a patient pose vector (PPN) from the image data representative of the patient, the patient pose vector (PPN) having an orientation that points toward a certain anatomical direction of the patient in a coordinate system of the patient represented by the image data.

5. The computer program product of claim 4, wherein the instructions are executable by the one or more processors to:
determine the orientation of the implant pose vector (IPN) relative to the coordinate system of the patient represented by the image data;
detect that the implant pose vector (IPN) and the patient pose vector (PPN) are oppositely directed; and
in response to detecting that the implant pose vector (IPN) and the patient pose vector (PPN) are oppositely directed, automatically reorient the representation of the model (190M, 190R) of the asymmetrical implant (190) to align the orientation of the implant pose vector (IPN) with the orientation of the patient pose vector (PPN).

6. The computer program product of claim 5, wherein the patient pose vector (PPN) is a posterior vector that has an orientation that points toward a posterior side of the patient in the coordinate system of the patient.

7. The computer program product of any preceding claim, wherein the instructions are executable by the one or more processors to present, on the display device (120), the representation (170M, 170R) of the implant insertion instrument (170) relative to a representation of the patient based on the tracking data.

8. The computer program product of claim 1, wherein the instructions are executable by the one or more processors to define a patient pose vector (PPN) relative to the image data based on the tracking data.

9. The computer program product of claim 8, wherein the instructions are executable by the one or more processors to determine the orientation of the implant pose vector (IPN) relative to the image data based on an expected spatial relationship between the implant pose vector (IPN) and the patient pose vector (PPN).

10. The computer program product of any one of claims 8 to 9, wherein the instructions are executable by the one or more processors to automatically align the orientation of the implant pose vector (IPN) and an orientation of the patient pose vector (PPN).

11. The computer program product of claim 1, wherein:
the orientation of the implant pose vector (IPN) is realized as a first orientation of the implant pose vector (IPN) relative to a z-vector of the representation (170M, 170R) of the implant insertion instrument (170); and
the instructions are executable by the one or more processors to present, on the display device (120), the representation of the model (190M, 190R) of the asymmetrical implant (190) in the first orientation relative to the representation (170M, 170R) of the implant insertion instrument (170) as the implant insertion instrument (170) is moved relative to the patient.

12. The computer program product of claim 11, wherein the instructions are executable by the one or more processors to:
determine an alignment metric based on the implant pose vector (IPN) and a patient pose vector (PPN) as the implant insertion instrument (170) is moved relative to the patient, wherein the alignment metric is an angle formed by the implant pose vector (IPN) and the patient pose vector (PPN);
determine a second orientation of the implant pose vector (IPN) relative to the z-vector of the representation (170M, 170R) of the implant insertion instrument (170) which is different from the first orientation, wherein the second orientation of the implant pose vector (IPN) is rotated about an x-vector of the representation (170M, 170R) of the implant insertion instrument (170) relative to the first orientation of the implant pose vector (IPN); and
present, on the display device (120), the representation of the model (190M, 190R) of the asymmetrical implant (190) in the second orientation relative to the representation (170M, 170R) of the implant insertion instrument (170) based on the alignment metric.

13. The computer program product of claim 12, wherein the instructions are executable by the one or more processors to:
compare the alignment metric to a misalignment threshold;
select one of the first orientation and the second orientation based on the comparison; and
present, on the display device (120), the representation of the model (190M, 190R) of the asymmetrical implant (190) in the selected one of the first orientation and the second orientation.

14. A computer-implemented method (200) for presenting an asymmetrical implant (190) relative to image data representative of a patient, wherein the asymmetrical implant (190) is configured to be attached to an implant insertion instrument (170) in one of multiple orientations, the computer-implemented method (200) comprising:
receiving (204) the image data representative of the patient;
receiving (208) tracking data indicative of a pose of the patient and a pose of the implant insertion instrument (170);
receiving (212) a model (190M, 190R) of the asymmetrical implant (190) which includes an implant pose vector (IPN);
determining (224) an orientation of the implant pose vector (IPN) relative to the image data based on the tracking data; and
presenting (228) a representation of the model (190M, 190R) of the asymmetrical implant (190) relative to a representation (170M, 170R) of the implant insertion instrument (170) based on the determined orientation of the implant pose vector (IPN).

15. A surgical system (100) comprising:
an implant insertion instrument (170) configured to attach to an asymmetrical implant (190) in one of multiple orientations;
a localizer (112) configured to track the implant insertion instrument (170) and a patient;
a display device (120); and
a computing device (140) coupled to the localizer (112) and the display device (120) and configured to:
obtain image data representative of the patient;
receive, from the localizer (112), tracking data indicative of a pose of the patient and a pose of the implant insertion instrument (170);
receive a model (190M, 190R) of the asymmetrical implant (190) which includes an implant pose vector (IPN);
determine an orientation of the implant pose vector (IPN) relative to the image data based on the tracking data; and
present, on the display device (120), a representation of the model (190M, 190R) of the asymmetrical implant (190) relative to a representation (170M, 170R) of the implant insertion instrument (170) based on the determined orientation of the implant pose vector (IPN).
